(19) Europäisches Patentamt
European Patent Office
Office européen des brevets

(11) **EP 1 073 676 B1**

(12) **EUROPEAN PATENT SPECIFICATION**

(45) Date of publication and mention
of the grant of the patent:
**22.06.2005 Bulletin 2005/25**

(51) Int Cl.⁷: **C07K 14/155**, G01N 33/569,
G01N 33/531

(21) Application number: **99920785.5**

(22) Date of filing: **26.04.1999**

(86) International application number:
**PCT/EP1999/002810**

(87) International publication number:
**WO 1999/057141 (11.11.1999 Gazette 1999/45)**

(54) **IMMUNODIAGNOSTIC REAGENT FOR THE DETECTION OF MAEDI-VISNA VIRUS AND CAEV INFECTION**

IMMUNODIAGNOSTISCHER WIRKSTOFF ZUM NACHWEISS VON MAEDI-VISNA VIRUS UND CAEV INFEKTIONEN

REACTIF D'IMMUNODIAGNOSTIC POUR LA DETECTION D'UNE INFECTION PAR LE VIRUS MAEDI-VISNA ET LE VIRUS ARTHRITE-ENCEPHALITE CAPRINE

(84) Designated Contracting States:
**AT BE CH CY DE DK ES FI FR GB GR IE IT LI LU MC NL PT SE**
Designated Extension States:
**AL LT LV MK RO SI**

(30) Priority: **30.04.1998 EP 98870101**

(43) Date of publication of application:
**07.02.2001 Bulletin 2001/06**

(73) Proprietor: **INNOGENETICS N.V.**
**9052 Ghent (BE)**

(72) Inventors:
• **GONZALEZ, Lorenzo**
**Peebles EH45 8EN (GB)**
• **TOLARI, Francesco**
**I-56124 Pisa (IT)**
• **WATT, Neil**
**Edinburgh EH12 8RB (GB)**
• **HARKISS, Gordon**
**Edinburgh EH9 3HE (GB)**
• **AMORENA, Beatriz**
**E-50008 Zaragoza (ES)**
• **SAMAN, Eric**
**B-2880 Bornem (BE)**
• **VAN EYNDE, Geertrui**
**B-9000 Gent (BE)**
• **BADIOLA DIEZ, Juan José**
**E-50720 La Cartuja Baja (ES)**

• **EXTRAMIANA, Belén**
**E-48902 Baracaldo (ES)**
• **BOLLO, Enrico**
**I-10040 Valdellatorre (IT)**
• **JUSTE, Ramon, A.**
**E-48180 Loiu. (ES)**

(56) References cited:
**EP-A- 0 344 578          EP-A- 0 379 949**
**WO-A-96/30527          FR-A- 2 586 427**
**FR-A- 2 721 618**

• **BERTONI E.A.: "Antibody reactivity to the immunodominant epitopes of the CAEV virus gp38 transmembrane protein associates with the development of arthritis" J.VIROL., vol. 68, no. 11, November 1994 (1994-11), pages 7139-7147, XP002084047 cited in the application**
• **IVANOV E.A.: "Effective method for synthetic peptide immobilization that increases the sensitivity and specificity of ELISA procedures" J.IMMUNOL.METHODS., vol. 153, 1992, pages 229-233, XP002084048**
• **ZANONI E.A.: "Expression in E.coli and sequencing of the coding region for the capsid protein of Dutch Maedi-Visna virus strain ZZV 1050:" J.CLIN.MICROBIOL., vol. 29, no. 7, July 1991 (1991-07), pages 1290-1294, XP002084049 cited in the application**

**Description**

The current invention relates to the field of Maedi-Visna virus (MVV) infection in mammals, more particularly in sheep, and the detection thereof. The invention relates to specific peptides useful in the immunodiagnosis of MVV infection, and to methods and kits using said peptides. The invention also relates to peptides, methods and kits, for the detection of CAEV infection in goats.

BACKGROUND OF THE INVENTION

Maedi-Visna virus (MVV) (or ovine lentivirus) is a non-oncogenic, exogenous retrovirus of the *Lentiviridae* subfamily (Cutlip and Laird, 1976), related to the human immune deficiency virus (HIV). MVV causes a disease in sheep that is characterized by a relatively long asymptomatic period in which the virus persists in the presence of a strong humoral and cellular response. Following a variable incubation period (2-10 years) the virus causes a chronic, progressive and fatal degenerative and inflammatory disease of the lungs, joints, mammary glands and central nervous system of sheep (Bulgin, 1990). Infected sheep are also immunocompromised and susceptible to secondary infections (Myer et al. 1988).

Most sheep respond to MVV infection by production of non-protective but detectable serum antibodies against viral proteins, especially viral envelope and capsid structural proteins. Several serological diagnostic tests have been developed to detect these antibodies, but only two methods, the agar gel immunodiffusion test (AGIDT) and the whole virus (WV) ELISA, are practical and routinely utilised (Cutlip et al. 1977; Houwers et al. 1982; Houwers and Schaake, 1987; Hoff-Jorgensen, 1990; Simard and Briscoe, 1990). Good estimates for the sensitivity and specificity of the AGIDT and WV-ELISA are not available, largely due to the lack of an adequate "gold standard" MVV diagnostic procedure (Campbell et al. 1994). In addition to the poorly defined sensitivity and specificity, a major problem for both the AGIDT and WV-ELISA is the generally low quality (i.e. low purity and heterogenous composition) and high expense of the viral test antigens employed. Viral antigens for conventional assays are produced from whole virion lysates prepared from time consuming, expensive and tedious MVV propagation in cell culture systems. Specific viral protein yield is low, while purification is difficult and frequently results in co-purification of undefined host cellular proteins. Thus, false-positive (non-specific) reactions may occur owing to cross reactions with mammalian cell proteins and false-negative reactions may result from the absence or insufficient quantity of specific immunogenic viral proteins in the test antigen preparation. Furthermore, the AGIDT and WV-ELISA are not suitable for reproducible large scale automated testing, precluding an economic and efficient throughput of large numbers of samples, a prerequisite for efficient flock control.

Caprine arthritis-encephalitis virus (CAEV) is genetically and antigenically closely related to MVV. CAEV infection in goat is widespread, and may cause important economic losses. The main clinical symptoms are arthritis, chronic subclinical mastitis, and interstitial pneumonia. Encephalitis is a rare feature of CAEV infection in young goats. Similar to MVV infection in sheep, the laboratory diagnosis of CAEV infection is based on the demonstration of antiviral antibodies in agar gel immunodiffusion assays or enzyme linked immunosorbent assays (ELISA). MVV antigens are frequently used for the diagnosis of MVV-infection in sheep as well as for the diagnosis of CAEV-infection in goats (Coackley and Smith, 1984; Houwers and Schaake, 1987; Zwahlen et al., 1983).

Recently, recombinant viral proteins have been widely used for detection of antiviral antibodies, especially in human and veterinary lentivirology. Recombinant DNA techniques can efficiently yield large quantities of highly purified viral proteins. Among the recombinant antigens used for detection of anti-MVV antibody are those derived from core (gag) proteins (more particularely p25), the transmembrane glycoprotein (TM) and external envelope glycoprotein (Kwang and Cutlip, 1992a,b; Kwang et al. 1995; Power et al. 1995; Keen et al. 1995, FR 2 586 427). Boshoff et al. (1997) recently reported the use of a GST-TM-p25 fusion protein for detection of antibodies against MVV. The results presented, whilst encouraging, should be regarded as preliminary in view of the limited number of sheep tested. Moreover, the authors mention the possible reactivity of the (uncleaved) GST-part in the fusion protein with sheep sera, possibly causing problems of specificity.

Kwang and Torres (1994) describe an oligopeptide based immunoassay for the detection of antibodies against MVV in sheep. According to computer prediction models they identified three antigenic sites in the N-terminal segment of the MVV TM (gp46) protein, on the basis of which three peptides where synthesized. The synthetic peptides showed a very high specificity (no false positive reactions) when tested with a collection of known seropositive and seronegative sheep sera. However, the reaction sensitivity was lower than the corresponding recombinant TM protein, even when a combination of the three peptides was used. The authors conclude that recombinant proteins are the preferred reagents for development of MVV immunodiagnostics.

From the above, it seems that there is a continuing need for better immunodiagnostic methods for MVV detection in sheep, showing better characteristics of specificity, sensitivity, and reproducability. Moreover, there is a need for tests which would be more easy to produce, and feasible for automatisation.

It is an aim of the current invention to provide for peptides suitable for the immunodiagnosis of MVV infection, and the closely related CAEV infection.

It is also an aim of the present invention to provide for a combination of recombinant proteins and peptides for use in the immunodiagnosis of MVV infection, and the closely related CAEV infection.

The present invention also aims at new methods and kits for the immunodiagnosis of MVV infection, and the closely related CAEV infection.

All these aims have been met by the embodiments as set out hereafter.

The current invention particularly describes for a peptide having the following amino acid sequence (single letter amino acid code):

$$\text{QELDCWHYQHYCVTSTKS (SEQ ID NO 1)}$$
$$5 \qquad 10 \qquad 15$$

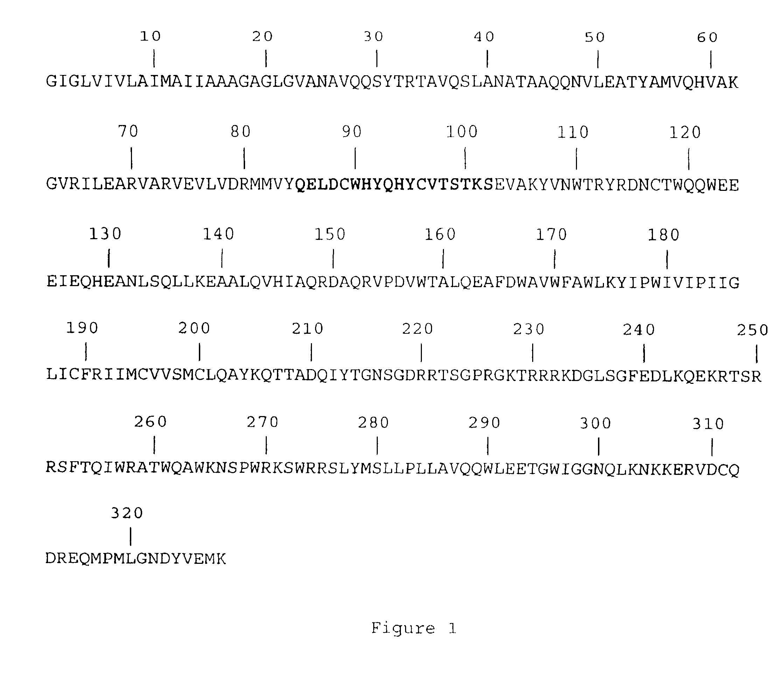

The amino acid sequence represented by SEQ ID NO 1 is part of the MVV transmembrane envelope glycoprotein (gp46), which from now on will be termed TM-antigen. The peptide is located from amino acid residue 85 to 102 in the full sequence of the gp46-protein as represented in figure 1, and encompasses the conserved Cys-Cys loop structure as described in related lentiviruses. The sequence of the MVV gp46-protein as shown in figure 1 is derived from the published sequence of the MVV E1 strain (Sargan et al. 1991). It should be understood that the TM sequence may vary slightly depending on the virus strain from which it is isolated. Especially conservative amino acid substitutions, as illustrated in Table 1 below, may occur frequently.

TABLE 1:    Overview of conservative amino acid substitutions.

| Amino acid | Conservative substitution by |
|---|---|
| Ser (S) | Thr, Gly, Asn |
| Arg (R) | His, Lys, Glu, Gln |
| Leu (L) | Ile, Met, Phe, Val, Tyr |
| Pro (P) | Ala, Thr, Gly |
| Thr (T) | Pro, Ser, Ala, Gly, His, Gln |
| Ala (A) | Pro, Gly, Thr |
| Val (V) | Met, Ile, Tyr, Phe, Leu |
| Gly (G) | Ala, Thr, Pro, Ser |
| Ile (I) | Met, Leu, Phe, Val, Tyr |
| Phe (F) | Met, Tyr, Ile, Leu, Trp, Val |
| Tyr (Y) | Phe, Trp, Met, Ile, Val, Leu |
| Cys (C) | Ser, Thr, Met |
| His (H) | Gln, Arg, Lys, Glu, Thr |
| Gln (Q) | Glu, His, Lys, Asn, Thr, Arg |
| Asn (N) | Asp, Ser, Gln |
| Lys (K) | Arg, Glu, Gln, His |
| Asp (D) | Asn, Glu, Gln |
| Glu (E) | Gln, Asp, Lys, Asn, His, Arg |
| Met (M) | Ile, Leu, Phe, Val |

Table 2 below shows some variations in the amino acid sequence of the peptide , which have been shown to occur among different natural isolates of MVV and CAEV strains.

**Table 2:** Natural sequence variations of the region in the TM protein encompassing the conserved Cys-Cys loop, as occurring in different MVV and CAEV isolates.

| Peptide sequence | SEQ ID NO | Strain* |
|---|---|---|
| QELDCWHYQHYCVTSTKS | 1 | MVV "EV1" |
| QELDCWHYQHYCVTSTRS | 2 | MVV "1514" |
| QELDCWHYQHYCVTSTRS | 3 | MVV "KV1772" |
| HELDCWHYQHYCVTSTKS | 4 | MVV "KM1071" |
| HELDCWHYQHYCVTSTRS | 5 | MVV "icelandic 1514" |
| HELDCWHYQHYCVTSTRS | 6 | MVV "1514" (LV1-1KS2) |
| HELDCWHYQHYCVTSTRT | 7 | MVV "SA-OMVV" |
| QELDCWHYHQYCITSTKT | 8 | CAEV "Clemens" |
| QELDCWHYHQYCITSTKT | 9 | CAEV "Cork" |
| QELDCWHYHQYCVTSTRA | 10 | CAEV "63" |
| QELDCWHYHQYCITSTRT | 11 | CAEV "TO-87" |

\* origin of the sequences (database and accession number):

| | |
|---|---|
| MVV strain EV1: | PIR JQ1165 |
| MVV strain 1514: | Swiss-Prot P03379 |
| MVV strain KV1772: | Swiss-Prot P35954 |
| MVV strain KM1071: | Genbank U51910 |
| MVV strain "Icelandic 1514": | Genbank M60609 |
| MVV strain 1514/clone LV1-1KS2: | Swiss Prot P23423 |
| MVV strain SA-OMVV: | Swiss-Prot P16899 |
| CAEV strain "Clements": | Genbank M33677 |
| CAEV strain Cork: | Swiss Prot P31626 |
| CAEV strain 63: | PIR VCLJC6 |
| CAEV strain TO-87: | Genbank S80269 |

From the above alignment it is apparent that especially positions 1, 9, 10, 13, 1 7 and 18 of the peptide are prone to conservative substitions. A more general formula for the peptide is therefore represented below:

$$X_1 ELDCWHYX_9X_{10}YCX_{13}TSTX_{17}X_{18} \qquad \text{(SEQ ID NO 12)}$$

wherein

- $X_1$ represents Q or a conservative substitution of Q, preferably H,
- $X_9$ represents Q or a conservative substitution of Q, preferably H,
- $X_{10}$ represents H or a conservative substitution of H, preferably Q,
- $X_{13}$ represents V or a conservative substitution of V, preferably I,
- $X_{17}$ represents K or a conservative substitution of K, preferably R,
- $X_{18}$ represents S or a conservative substitution of S, preferably T or A.

It is noted that in the majority of the cases of MVV sequences, $X_9$ is Q and $X_{10}$ is H, while in the majority of CAEV sequences, it is vice versa ($X_9$ is H and $X_{10}$ is Q).

Several studies in MVV related lentiviruses, such as HIV (Human Immunodeficiency Virus), SIV (Simian Immunodeficiency Virus) and CAEV have shown that the conserved Cys-Cys loop region in the TM protein constitutes an immunodominant region (reviewed by Pancino et al. 1994). In particular, a CAEV peptide with a sequence homologous to the sequence represented by SEQ ID NO 1 has been described as being reactive with sera from CAEV infected goat (Bertoni et al. 1994, WO 96/00784 FR 2721618).

As will be shown further in the examples section, the current invention demonstrates that, quite unexpectedly, the peptide represented by SEQ ID NO 1 is not immunoreactive when bound as such on the solid phase of an immunoassay. Only when the peptide is biotinylated, and the binding to the solid phase occurs via the interaction of the biotin moiety with streptavidin or avidin, the peptide is found to be immunoreactive with sera from MVV infected sheep.

The invention thus describes more particularly for a biotinylated peptide represented by the following formula:

$$[B_1 - L_1] - X_1 ELDCWHYX_9 X_{10} YCX_{13} TSTX_{17} X_{18} - [L_2 - B_2]$$  (SEQ ID NO 13)

wherein

- X$_1$ represents Q or a conservative substitution of Q, preferably H,
- X$_9$ represents Q or a conservative substitution of Q, preferably H,
- X$_{10}$ represents H or a conservative substitution of H, preferably Q,
- X$_{13}$ represents V or a conservative substitution of V, preferably I,
- X$_{17}$ represents K or a conservative substitution of K, preferably R,
- X$_{18}$ represents S or a conservative substitution of S, preferably T or A,
- B represents a biotinyl moiety,
- L represents an amide bond or an amino acid linker sequence containing from 1 to 5 amino acid residues, and with either [B$_1$ -L$_1$] or [L$_2$ -B$_2$ ] being present or both.

Linker sequences result in a more spacial presentation of the peptide epitope on the solid phase, and consequently in a more efficient recognition of the peptide by antibodies. Frequenlty used amino acids for the construction of linker sequences are a.o. glycine, alanine, beta-alanine, lysine, epsilon-lysine, aminocapronic acid, ornithine, aminobutyric acid. It has to be understood that the biotinyl moiety is attached via its carboxyl group to an amino group of the amino acids of the linker sequence or of the peptide. This amino group may be in alpha position, or located in the side chain of the respective amino acid (e.g. epsilon lysine). The linker sequence illustrated in the examples section of the present invention consists of a number of glycine residues, preferably 2 glycines. However, it should be understood that other linker sequences currently used in the art of peptide chemistry may be as efficient.

The core sequence determining the immunoreactivity of the peptides described above is embraced by the two cysteine residues. The following truncated peptides may be considered as equivalents of the peptides

$$[B_1 - L_1] - ELDCWHYX_9 X_{10} YCX_{13} TSTX_{17} X_{18} - [L_2 - B_2]$$  (SEQ ID NO 14)

$$[B_1 - L_1] - LDCWHYX_9 X_{10} YCX_{13} TSTX_{17} X_{18} - [L_2 - B_2]$$  (SEQ ID NO 15)

$$[B_1 - L_1] - DCWHYX_9 X_{10} YCX_{13} TSTX_{17} X_{18} - [L_2 - B_2]$$  (SEQ ID NO 16)

$$[B_1 - L_1] - CWHYX_9 X_{10} YCX_{13} TSTX_{17} X_{18} - [L_2 - B_2]$$  (SEQ ID NO 17)

$$[B_1 - L_1] - X_1 ELDCWHYX_9 X_{10} YCX_{13} TSTX_{17} - [L_2 - B_2]$$  (SEQ ID NO 18)

$$[B_1 - L_1] - ELDCWHYX_9 X_{10} YCX_{13} TSTX_{17} - [L_2 - B_2]$$  (SEQ ID NO 19)

$$[B_1 - L_1] - LDCWHYX_9 X_{10} YCX_{13} TSTX_{17} - [L_2 - B_2]$$  (SEQ ID NO 20)

$$[B_1 - L_1] - DCWHYX_9 X_{10} YCX_{13} TSTX_{17} - [L_2 - B_2]$$  (SEQ ID NO 21)

$[B_1 -L_1] -CWHYX_9X_{10}YCX_{13}TSTX_{17} - [L_2- B_2]$ (SEQ ID NO 22)

$[B_1 -L_1] -X_1ELDCWHYX_9X_{10}YCX_{13}TST - [L_2- B_2]$ (SEQ ID NO 23)

$[B_1 -L_1] -ELDCWHYX_9X_{10}YCX_{13}TST - [L_2- B_2]$ (SEQ ID NO 24)

$[B_1 -L_1] -LDCWHYX_9X_{10}YCX_{13}TST - [L_2- B_2]$ (SEQ ID NO 25)

$[B_1 -L_1] -DCWHYX_9X_{10}YCX_{13}TST - [L_2- B_2]$ (SEQ ID NO 26)

$[B_1 -L_1] -CWHYX_9X_{10}YCX_{13}TST - [L_2- B_2]$ (SEQ ID NO 27)

$[B_1 -L_1] -X_1ELDCWHYX_9X_{10}YCX_{13}TS - [L_2- B_2]$ (SEQ ID NO 28)

$[B_1 -L_1] -ELDCWHYX_9X_{10}YCX_{13}TS - [L_2- B_2]$ (SEQ ID NO 29)

$[B_1 -L_1] -LDCWHYX_9X_{10}YCX_{13}TS - [L_2- B_2]$ (SEQ ID NO 30)

$[B_1 -L_1] -DCWHYX_9X_{10}YCX_{13}TS - [L_2- B_2]$ (SEQ ID NO 31)

$[B_1 -L_1] -CWHYX_9X_{10}YCX_{13}TS - [L_2- B_2]$ (SEQ ID NO 32)

$[B_1 -L_1] -X_1ELDCWHYX_9X_{10}YCX_{13}T - [L_2- B_2]$ (SEQ ID NO 33)

$[B_1 -L_1] -ELDCWHYX_9X_{10}YCX_{13}T - [L_2- B_2]$ (SEQ ID NO 34)

$[B_1 -L_1] -LDCWHYX_9X_{10}YCX_{13}T - [L_2- B_2]$ (SEQ ID NO 35)

$[B_1 -L_1] -DCWHYX_9X_{10}YCX_{13}T - [L_2- B_2]$ (SEQ ID NO 36)

$[B_1 -L_1] -CWHYX_9X_{10}YCX_{13}T - [L_2- B_2]$ (SEQ ID NO 37)

$[B_1 -L_1] -X_1ELDCWHYX_9X_{10}YCX_{13} - [L_2- B_2]$ (SEQ ID NO 38)

$[B_1 -L_1] -ELDCWHYX_9X_{10}YCX_{13} - [L_2- B_2]$ (SEQ ID NO 39)

$[B_1 -L_1] -LDCWHYX_9X_{10}YCX_{13} - [L_2- B_2]$ (SEQ ID NO 40)

$[B_1 -L_1]$ -DCWHYX$_9$X$_{10}$YCX$_{13}$ - $[L_2- B_2]$ (SEQ ID NO 41)

$[B_1 -L_1]$ -CWHYX$_9$X$_{10}$YCX$_{13}$ - $[L_2- B_2]$ (SEQ ID NO 42)

$[B_1 -L_1]$ -X$_1$ELDCWHYX$_9$X$_{10}$YC - $[L_2- B_2]$ (SEQ ID NO 43)

$[B_1 -L_1]$ -ELDCWHYX$_9$X$_{10}$YC - $[L_2- B_2]$ (SEQ ID NO 44)

$[B_1 -L_1]$ -LDCWHYX$_9$X$_{10}$YC - $[L_2- B_2]$ (SEQ ID NO 45)

$[B_1 -L_1]$ -DCWHYX$_9$X$_{10}$YC - $[L_2- B_2]$ (SEQ ID NO 46)

$[B_1 -L_1]$ -CWHYX$_9$X$_{10}$YC - $[L_2- B_2]$ (SEQ ID NO 47)

wherein

- X$_1$ represents Q or a conservative substitution of Q, preferably H,
- X$_9$ represents Q or a conservative substitution of Q, preferably H,
- X$_{10}$ represents H or a conservative substitution of H, preferably Q,
- X$_{13}$ represents V or a conservative substitution of V, preferably I,
- X$_{17}$ represents K or a conservative substitution of K, preferably R,
- X$_{18}$ represents S or a conservative substitution of S, preferably T or A,
- B represents a biotinyl moiety,
- L represents an amide bond or an amino acid linker sequence containing from 1 to 5 amino acid residues, and with either $[B_1 -L_1]$ or $[L_2 -B_2]$ being present or both.

Further down in the examples section it is shown that the MVV peptides of the current invention can be perfectly used for the detection of CAEV infection in goats, besides their demonstrated usefulness as reagents for the detection of MVV infection in sheep.
The terms "MVV-derived" and "CAEV-derived" peptides implies that the sequences of the respective peptides are part of a protein sequence isolated from a MVV, respectively CAEV strain. As stated above, MVV-derived and CAEV-derived peptides may show extensive crossreactivity, when it comes to the detection of MVV infection in sheep, resp. CAEV infection in goat.
Specific MVV-derived peptides are represented by the following family of sequences:

$[B_1 -L_1]$ - X$_1$ELDCWHYQHYCVTSTX$_{17}$X$_{18}$ - $[L_2- B_2]$ (SEQ ID NO 48)

$[B_1 -L_1]$ - ELDCWHYQHYCVTSTX$_{17}$X$_{18}$ - $[L_2- B_2]$ (SEQ ID NO 49)

$[B_1 -L_1]$ - LDCWHYQHYCVTSTX$_{17}$X$_{18}$ - $[L_2- B_2]$ (SEQ ID NO 50)

$[B_1 -L_1]$ - DCWHYQHYCVTSTX$_{17}$X$_{18}$ - $[L_2- B_2]$ (SEQ ID NO 51)

$[B_1 -L_1]$ - CWHYQHYCVTSTX$_{17}$X$_{18}$ - $[L_2- B_2]$ (SEQ ID NO 52)

$[B_1 - L_1] - X_1ELDCWHYQHYCVTSTX_{17} - [L_2 - B_2]$ (SEQ ID NO 53)

$[B_1 - L_1] - ELDCWHYQHYCVTSTX_{17} - [L_2 - B_2]$ (SEQ ID NO 54)

$[B_1 - L_1] - LDCWHYQHYCVTSTX_{17} - [L_2 - B_2]$ (SEQ ID NO 55)

$[B_1 - L_1] - DCWHYQHYCVTSTX_{17} - [L_2 - B_2]$ (SEQ ID NO 56)

$[B_1 - L_1] - CWHYQHYCVTSTX_{17} - [L_2 - B_2]$ (SEQ ID NO 57)

$[B_1 - L_1] - X_1ELDCWHYQHYCVTST - [L_2 - B_2]$ (SEQ ID NO 58)

$[B_1 - L_1] - ELDCWHYQHYCVTST - [L_2 - B_2]$ (SEQ ID NO 59)

$[B_1 - L_1] - LDCWHYQHYCVTST - [L_2 - B_2]$ (SEQ ID NO 60)

$[B_1 - L_1] - DCWHYQHYCVTST - [L_2 - B_2]$ (SEQ ID NO 61)

$[B_1 - L_1] - CWHYQHYCVTST - [L_2 - B_2]$ (SEQ ID NO 62)

$[B_1 - L_1] - X_1ELDCWHYQHYCVTS - [L_2 - B_2]$ (SEQ ID NO 63)

$[B_1 - L_1] - ELDCWHYQHYCVTS - [L_2 - B_2]$ (SEQ ID NO 64)

$[B_1 - L_1] - LDCWHYQHYCVTS - [L_2 - B_2]$ (SEQ ID NO 65)

$[B_1 - L_1] - DCWHYQHYCVTS - [L_2 - B_2]$ (SEQ ID NO 66)

$[B_1 - L_1] - CWHYQHYCVTS - [L_2 - B_2]$ (SEQ ID NO 67)

$[B_1 - L_1] - X_1ELDCWHYQHYCVT - [L_2 - B_2]$ (SEQ ID NO 68)

$[B_1 - L_1] - ELDCWHYQHYCVT - [L_2 - B_2]$ (SEQ ID NO 69)

$[B_1 - L_1] - LDCWHYQHYCVT - [L_2 - B_2]$ (SEQ ID NO 70)

$[B_1 - L_1] - DCWHYQHYCVT - [L_2 - B_2]$ (SEQ ID NO 71)

$[B_1 -L_1]$ - CWHYQHYCVT - $[L_2- B_2]$      (SEQ ID NO 72)

$[B_1 -L_1]$ - $X_1$ELDCWHYQHYCV - $[L_2- B_2]$      (SEQ ID NO 73)

$[B_1 -L_1]$ - ELDCWHYQHYCV - $[L_2- B_2]$      (SEQ ID NO 74)

$[B_1 -L_1]$ - LDCWHYQHYCV - $[L_2- B_2]$      (SEQ ID NO 75)

$[B_1 -L_1]$ - DCWHYQHYCV - $[L_2- B_2]$      (SEQ ID NO 76)

$[B_1 -L_1]$ - CWHYQHYCV - $[L_2- B_2]$      (SEQ ID NO 77)

$[B_1 -L_1]$ - $X_1$ELDCWHYQHYC - $[L_2- B_2]$      (SEQ ID NO 78)

$[B_1 -L_1]$ - ELDCWHYQHYC - $[L_2- B_2]$      (SEQ ID NO 79)

$[B_1 -L_1]$ - LDCWHYQHYC - $[L_2- B_2]$      (SEQ ID NO 80)

$[B_1 -L_1]$ - DCWHYQHYC - $[L_2- B_2]$      (SEQ ID NO 81)

$[B_1 -L_1]$ - CWHYQHYC - $[L_2- B_2]$      (SEQ ID NO 82)

wherein

- $X_1$ represents Q or a conservative substitution of Q, preferably H,
- $X_{17}$ represents K or a conservative substitution of K, preferably R,
- $X_{18}$ represents S or a conservative substitution of S, preferably T or A,
- B represents a biotinyl moiety,
- L represents an amide bond or an amino acid linker sequence containing from 1 to 5 amino acid residues, and with either $[B_1 -L_1]$ or $[L_2 -B_2]$ being present or both.

The group of biotinylated peptides of the invention all originate from the same immunodominant region in the TM-protein (further called "TM-peptides").
The invention thus describes for biotinylated TM-peptides useful in the detection of MVV and CAEV infection, and represented by the following formula:

$[B_1 -L_1]$ - (a) - CWHY$X_9X_{10}$YC - (b)- $[L_2- B_2]$      (SEQ ID NO 83)

wherein B represents a biotinyl moiety and L represents an amide bond or an amino acid linker sequence containing from 1 to 5 amino acid residues, with either $[B_1 -L_1]$ or $[L_2 -B_2]$ present or both, and wherein (a) represents from none to the maximum number of contiguous amino acids located from position 1 to position 4 in the sequence represented by SEQ ID NO 1 2, and wherein (b) represents from none to the maximum number of contiguous amino acids located from position 13 to position 18 in the sequence represented by SEQ ID NO 12.
The group of MVV derived biotinylated TM-peptides useful for the detection of MVV and CAEV infection is represented by the following formula:

$$[B_1 - L_1] - (a) - CWHYQHYC - (b) - [L_2 - B_2] \qquad \text{(SEQ ID NO 84)}$$

wherein B represents a biotinyl moiety and L represents an amide bond or an amino acid linker sequence containing from 1 to 5 amino acid residues, with either $[B_1 - L_1]$ or $[L_2 - B_2]$ present or both at the same time, and wherein (a) represents from none to the maximum number of contiguous amino acids located from position 1 to position 4 in the sequence represented by SEQ ID NO 1 2, and wherein (b) represents from none to the maximum number of contiguous amino acids located from position 13 to position 18 in the sequence represented by SEQ ID NO 12.

The TM-peptide according to the invention is represented by the following sequence:

$$\text{B-GGQELDCWHYQHYCVTSTKS (SEQ ID NO 85)}$$

As will be shown in the examples section, the above described peptide (SEQ ID NO 85) shows an extremely good immunoreactivity with sera from MVV-infected sheep, when presented on a solid support via streptavidin or avidin interaction. In fact, the sensitivity of detection of immunoassays based on this peptide alone as a detection agent is at least 90%, in many cases 95%, and sometimes even approaching 100%.

The term "sensitivity" as used throughout the current invention means the percentage of sera from MVV-infected subjects which are showing a positive (= above background level) immunoreactivity with the peptide(s) of the invention.

The term "specificity" as used throughout the current invention means (the total number of healthy ( =originating from not MVV-infected subjects) sera minus the number of healthy sera which are showing a false positive immunoreactivity with the peptide(s) of the invention, divided by the total number of healthy sera) X 100%.

Truncated biotinylated TM-peptides, belonging to the above described family of sequences of SEQ ID NO 14 to 82 show a similar immunoreactivity with sera from MVV infected sheep as the preferred TM peptide represented by SEQ ID NO 85. The specificity of immunoassays based on those peptides is about the same, the sensitivity may be the same or somewhat lower (from 50% to 100%) as compared to the reactivity of the same sera with the peptide represented by SEQ ID NO 85.

The synthesis of oligopeptides may be carried out according to any method known in the art of peptide chemistry such as described for example in Bodanszky ( 1 993). As will be described further in the examples section, the peptides of the current invention have been synthesized by solid phase peptide synthesis using Fmoc-chemistry (Fmoc = 9-fluoromethyloxycarbonyl). Currently there are a number of apparatus available which enable a quick and fully automated peptide synthesis, such as for example the Symphony/Multiplex peptide synthesiser from Rainin Instruments Co. Inc.

The biotinylation process can be carried out according to methods well known in the art of peptide chemistry (see e.g. WO 93/18054). Attachment of the biotinyl moiety may be done during or after peptide synthesis. The biotinyl moiety may be attached N-terminally, C-terminally or internally.

The invention further describes for a biotinylated TM peptide as described above, more specifically a biotinylated TM peptide as represented by any of SEQ ID NO 13-85, whereby the two cysteine (C) residues present in the amino acid sequence are covalently linked to form an intramolecular cystine bridge. This chemically cyclized peptide mimics the loop structure which is thought to exist in the native TM protein.

The formation of the cystine bond may be carried out according to known methods in the art of protein chemistry. There are a number of oxidizing compounds or conditions which specifically induce the formation of disulfide bridges, such as for example air oxidation, $I_2$, $K_3Fe(CN)_6$ (McCurdy, 1989; Pohl et al. 1993).

The current invention also provides for a biotinylated TM-peptide as described above, whereby the biotin moiety is already complexed to a streptavidin or avidin molecule.

The current invention moreover provides for a biotinylated TM-peptide as described above, complexed to a streptavidin or avidin molecule, whereby the complex is already immobilized to a solid support.

The invention also provides for a peptide composition comprising a biotinylated peptide as described above, in combination with any other MVV peptide or recombinant protein.

A preferred peptide composition according to the present invention comprises a biotinylated TM-peptide as described above and a recombinant MVV *gag* (p25) polypeptide, or a fragment thereof.

The use of recombinant MVV *gag* protein in the serodiagnosis of MVV infection in sheep and CAEV infection in goats has already been described before (see e.g. Zanoni et al. 1991). However, sensitivity and specificity obtained with this recombinant antigen were not satisfactory. Moreover, the recombinant MVV *gag* protein as described by Zanoni et al. (1991) contained a GST (Glutathion-S-transferase) part to which sheep sera may bind aspecifically.

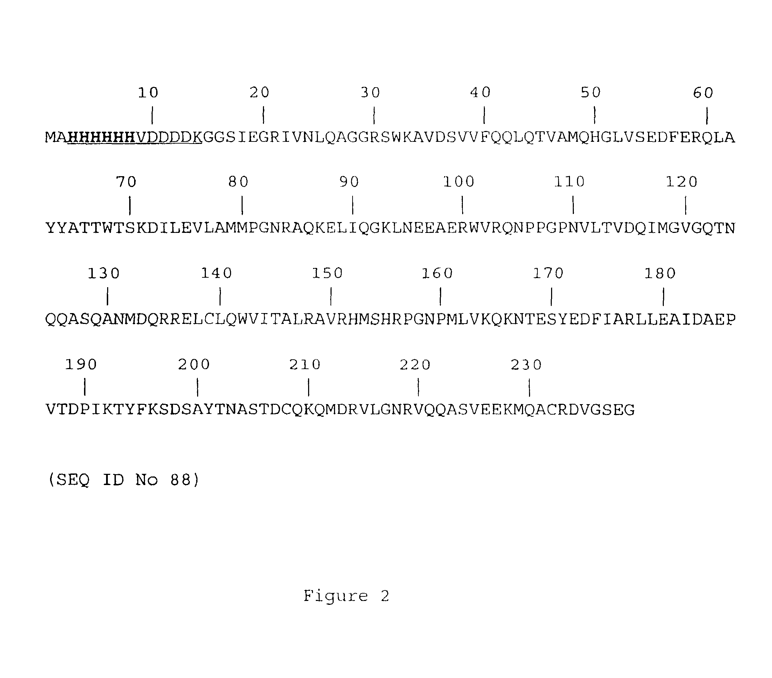

The current invention provides for a peptide composition comprising a biotinylated TM-peptide as described above and a recombinant MVV p25 protein as depicted in figure 2 (SEQ ID NO 88).

The amino acid sequence of the recombinant p25 protein as shown in figure 2 contains, in addition to the p25 protein sequence itself, a "His-tag" consisting of 6 histidine residues, allowing purification of the recombinant protein by IMAC (Immobilized Metal Ion Affinity Chromatography), and an enterokinase site enabling the cleavage of the His-tag after purification.

It is to be understood that the recombinant p25 protein as depicted in figure 2 only serves as an example, and does not exclude other recombinant MVV p25 proteins having a similar immunoreactivity. The sequence of the gene encoding the MVV p25 protein has been amply described in the prior art (e.g. for the MVV E 1 strain by Sargan et al. 1991). Using known techniques for cloning and recombinant expression, e.g. in a bacterial host such as *E. coli*, it is quite straightforward to obtain the recombinantly produced p25 protein, or a fragment thereof.

As shown further in the examples section, the combination of a biotinylated TM-peptide as described above with the MVV p25 recombinant protein, leads to an immunodiagnostic reagent detecting MVV-infected sheep with both a sensitivity and specificity approaching 100%. The same peptide composition may be used for the serodiagnosis of CAEV infection in goats.

The amino acid sequence of the peptides of the invention, may also be inserted in or added to a second amino acid sequence, resulting in a fusion protein. The second amino acid sequence may be originating either from a totally unrelated ( = heterologous) protein, which would function merely as a "carrier" protein of the peptide of the invention, or it may be originating from MVV. In the latter case, the second amino acid sequence may contain additional immunogenic regions suitable for detection of MVV infection (epitopes), leading to an increased sensitivity of the fusion protein as compared to the peptide alone. The second amino acid sequence of the fusion protein preferably corresponds to the MVV *gag* sequence or a part of it. The presentation of the MVV TM peptide's epitope via a fusion protein, may be similar to the presentation of the TM peptide via biotin/streptavidin interaction. In addition, the heterologous sequence in the fusion protein may bring about any desired side effect to the resulting fusion protein, e.g. it may optimize the expression, the purification, the immobilization on a surface etc.

The construction and expression of fusion proteins may be done according to well-known techniques in the art of recombinant DNA technology (such as illustrated by Maniatis et al. (1982)). The DNA fragments encoding the different parts of the fusion protein are linked together, such that the reading frame is not shifted. The hybrid DNA molecule is than operably linked to an expression cassette allowing the expression of the fusion protein in a host cell. Possible host cells for the expression of MVV recombinant proteins are bacteria (such as *E. coli*) or eukaryotic cells (yeasts or mammalian cell lines).

The expression "operably linked" refers to a juxtaposition wherein the components are configured so as to perform their usual function. Thus, control sequences operably linked to a coding sequence are capable of effecting the expression of the coding gene.

The term "expression cassette" encompasses a cassette of elements enabling the correct transcription and translation of the encoded sequences by the host cell machinery: i.e. a promoter sequence, a ribosome binding site, and possibly also regulating sequences, or secreting signals.

The above-described MVV TM-peptides or recombinant proteins may be immobilized to a solid support, by covalent or non-covalent linkage. A preferred way of immobilization for the biotinylated TM-peptides of the current invention is the coating by way of their biotin moiety to a streptavidin or avidin coated surface. The complexation of the biotinylated peptide to streptavidin (or avidin) may also occur prior to the binding, i.e. in solution, followed by the coating of the complex to the solid phase, as described furtheron in the examples section.

The invention thus also provides for a solid support upon which the above-mentioned biotinylated TM-peptide or peptide composition has been immobilized.

The solid support onto which the peptides or recombinant proteins of the invention are coated may be chosen from the supports commonly used in the art of immunodiagnosis. Depending on the type of assay the solid support may be a polystyrene carrier (microtiter plate (e.g. ELISA) or beads (e.g. microbeads assay)), a nylon membrane (LIA), latex beads (coagulation tests), etc.

According to another embodiment, the invention provides for a method for immunodetection of MVV or CAEV infection in mammals, said method comprising the steps of:

- contacting a biological sample from said mammal with a biotinylated TM peptide or a peptide composition as described above, under conditions allowing the formation of an immunological complex, and
- detecting the presence of an immunological complex formed between the biotinylated TM peptide or peptide composition and the anti-MVV or anti-CAEV antibodies which may be present in the sample.

It is to be understood that the presence of anti-MVV or anti-CAEV antibodies in a biological sample indicates the existence of an MVV-infection (resp. CAEV infection) in the mammal from which the sample was taken.

The term "mammal" may refer to a human individual, or any mammal animal. Preferably, the term mammal refers to sheep, the natural host of MVV, or goat, the natural host of CAEV. Although MVV has not been shown yet to occur in humans, it is not excluded that this may happen, as was recently shown for Caprine Arthritis Encephalitis Virus (CAEV) (see WO 97/33615).

The term "biological sample" refers to a sample of blood, serum, plasma, milk, urine, cerobrospinal fluid or any other body fluid which may contain antibodies against MVV. Preferably the biological sample is a serum sample.

According to another embodiment, the invention provides for a method for immunodetection of MVV or CAEV infection in mammals, said method comprising the steps of:

- contacting a biological sample from said mammal with a biotinylated TM peptide or a peptide composition or a fusion protein as described above, under conditions allowing the formation of an immunological complex, and
- detecting the presence of an immunological complex formed between the biotinylated TM peptide or peptide composition or the fusion protein and the anti-MVV or anti-CAEV antibodies which may be present in the sample.

The formation and detection of an antibody-antigen immunological complex, indicating the presence of anti-MVV or anti-CAEV antibodies in the sample, may be done according to methods well-known in the art.

Design of immunoassays suitable for detection of antibodies in a sample, is subject to a great deal of variation, and many formats are known in the art. Most assays involve the use of labeled antibody and/or peptide. The labels may be, for example, enzymatic (like horse radish peroxidase, alkaline phosphatase), fluorescent, chemoluminescent, radioactive, or dye molecules. In an enzyme linked immuno sorbent assay (ELISA) the label is enzymatic, and the detection signal is usually colorimetric (optical density).

Different types of ELISA may be feasable for detection of antibodies in a sample. In an indirect ELISA the peptides of the invention are coated on a solid surface (microtiter plates) where they capture the antibodies possibly present in the sample. The bound antibodies from the sample are subsequently detected by a labeled second antibody (conjugate). The signal generated by the latter is quantified. In an antigen sandwich ELISA, the peptides of the invention are used twice, first as a capturing agent (bound on the surface of e.g. a microtiter plate) and secondly as a detecting agent (labelled peptide reacting with the bound antibodies from the sample). The antibodies from the sample are "sandwiched" between two layers of peptide (this is made possible by the two antigen binding sites which are present on each antibody).

According to a preferred embodiment, the invention provides for an immunodetection method or assay as described above, wherein said method or assay is an ELISA.

Furthermore, the invention provides for an immunodiagnostic kit for the detection of MVV infection or CAEV infection in mammals , said kit comprising a biotinylated TM-peptide or peptide composition as described above.

In addition, the invention provides for an immunodiagnostic kit for the detection of MVV infection or CAEV infection in mammals , said kit comprising a biotinylated TM-peptide or peptide composition or fusion protein as described above.

In a preferred embodiment, the immunodiagnostic kit comprises a solid support onto which a biotinylated TM peptide of the invention has been readily immobilized, preferably by biotin/streptavidin interaction.

Alternatively, a preferred immunodiagnostic kit of the invention comprises as a reagent a biotinylated TM peptide of the invention in the format of a biotin/streptavidin complex.

The immunodiagnostic kits of the invention comprise, besides the biotinylated TM peptides, the necessary buffers and reagents for carrying out the assay.

In a preferred embodiment, the immunodiagnostic kit of the invention comprises a biotinylated TM peptide as described above, in combination with a recombinant p25 protein. Alternatively, the immunodiagnostic kit of the current invention comprises a fusion protein as described above.

FIGURE LEGEND

Figure 1

Amino acid sequence representing the TM protein of the MVV EV1 isolate (as described by Sargan et al. 1991). The sequence in bold corresponds to the TM1-peptide of the invention.

Figure 2

Amino acid sequence of the recombinant p25 protein described in the current invention (SEQ ID NO 88). Underlined and bold: His-tag

Underlined: enterokinase (EK) site

Figure 3

Physical map of the expression construct pIGRHISAMVVp25. Relevant restriction sites are indicated.

Figure 4

Induction experiment for the production of HisMVVp25 protein.

Samples were taken at different time intervals (lanes 1, 2, and 3 resp. uninduced, 1.5 h and 3 h induction) and analysed on SDS-PAGE.

Detection of proteins with coomassie staining. Distribution between insoluble (lane 4), inclusion bodies (lane 5) and soluble (lane 6) fraction is also shown. The arrow indicates the MVV fusion protein.

Figure 5

Analysis of the purified HisMVVp25 protein.

Part A: analysis of purified protein from the soluble fraction (lanes 3 and 4) and from the inclusion body fraction (lanes 1 and 2). Detection by Coomassie staining.

Part B: Western Blot analysis using a MVV infected sheep serum (left) or an anti-coli serum (right). Lanes 1 and 3, soluble fraction; lanes 2 and 4, inclusion bodies.

Figure 6

Indirect ELISA using MVV biotinylated TM1 peptide (SEQ ID NO 85) as detecting reagent. OD values correspond to those indicated in Table 4. Black bars represent OD values with TM1 peptide coated on the microtiter plate (directly coated or via streptavidin). White bars represent control values without TM1 peptide coated. The horizontal line represents the cutoff (c.o.) level.

Part 6A: direct coating of the biotinylated TM1 peptide

Part 6B: coating of the biotin/streptavidin complexed TM1 peptide

Sera used are classified according to their reacitivity in AGIDT:

| gp135+ | clearly positive in AGIDT with anti-gp135 reference antibody |
| gp135$_{+/-}$ | weakly positive in AGIDT with anti-gp135 reference antibody |
| p25 + | clearly positive in AGIDT with anti-p25 reference antibody |
| doubtful | indeterminate reactivity in AGIDT |
| negative sera (marked with *) | originate from Icelandic flocks which are certified free of MVV. |

Figure 7

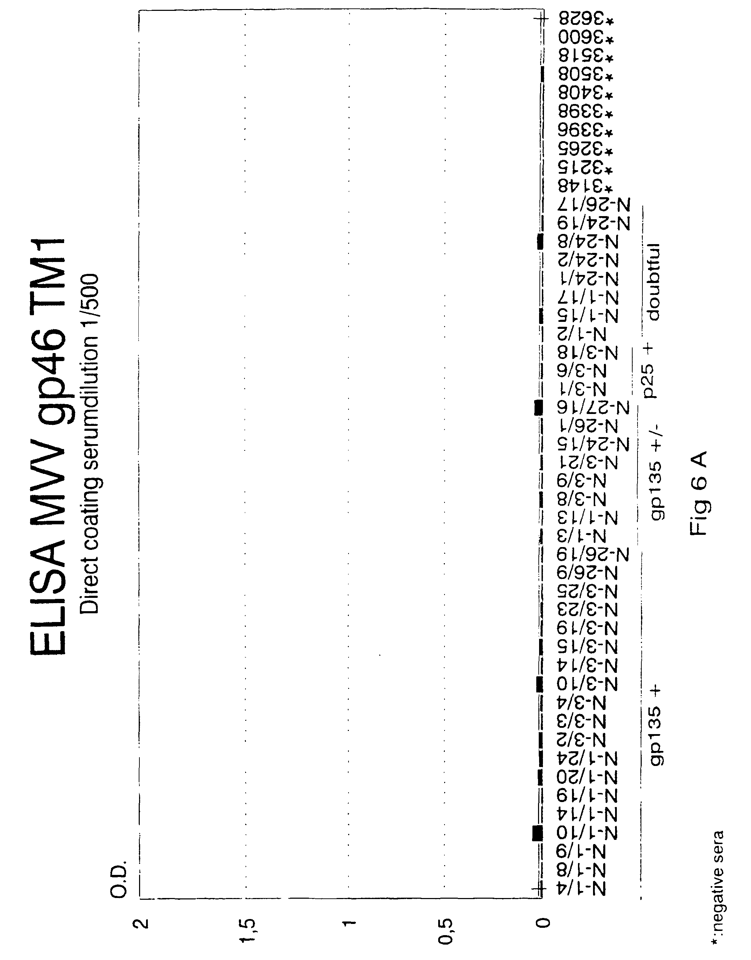

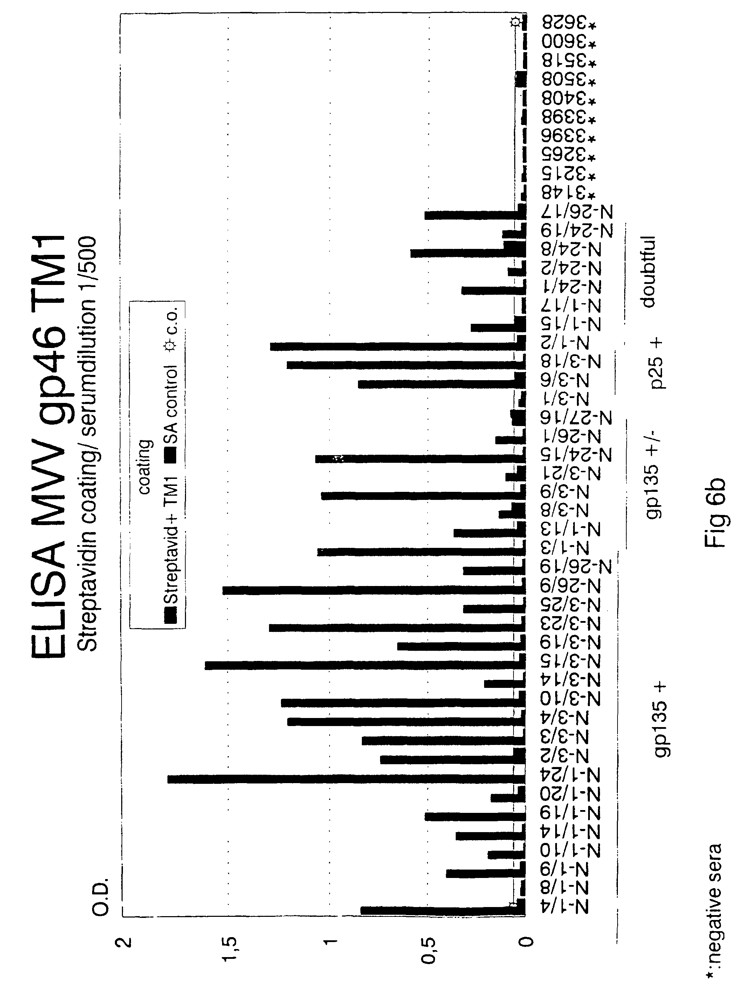

Comparative ELISA with the TM1, TM2 and TM3 peptides (resp. SEQ ID NO 85, 86, 87). The biotinylated peptides are complexed to streptavidin and subsequently coated on the microtiter plates. The same sera and reaction conditions are used as in figure 6. The vertical axis shows the S/N values (values > 1 are considered positive).

Figure 8

ELISA using recombinant p25 (SEQ ID NO 88) as detecting reagent. The same sera and reaction conditions are used as in figure 6. S/N values above 1 are considered to be positive.

Figure 9

"Combi ELISA" using a combination of biotinylated TM1 peptide (SEQ ID NO 85) coated via streptavidin interaction and recombinant p25 protein (SEQ ID NO 88) as detecting reagent. Same sera and same reaction conditions as in figure 6. Serum N1-17 was not tested in this series.

EXAMPLES

**Example 1 : Expression of recombinant MVV p25 protein in *E. coli***

*Cloning by PCR of the gene encoding the MVV p25 protein.*

The p25 protein of MVV is encoded by the *gag* precursor (group associated antigens) gene. This produces a non-glycosylated protein of 55 kD which is enzymatically cleaved and processed into p25 (the core protein), p16 (matrix protein) and p14 (the nucleoprotein). The core protein has been found to be the most immunogenic of all viral *gag* proteins. It is also the most conserved *gag* protein amongst all MVV isolates sequenced today.

The viral gene encoding the p25 protein has been cloned by PCR starting from unintegrated proviral DNA which was extracted from infected sheep fibroblasts, grown in vitro, by the modified Hirt method.

The following primer set was used for amplification of the p25 gene:

(a) 5'-TCCCGGGATCCATAGAAGGTAGAATTGTAAATCTGCAAGCAGG-3'

(sense) (SEQ ID NO 89)

(b) 5'-ACACCCGGGATCCCTTCTGATCCTACATCTC-3' (antisense) (SEQ ID NO 90)

The PCR obtained fragment was cloned into the pTZ vector (Pharmacia) and sequenced. It was then transferred as a *Bam*HI fragment to pBluescript SK (stratagene) and subsequently cloned as a Sma1/Xbal fragment into the expression vector pIGRHISA.

*Expression of the p25 antigen in E. coli*

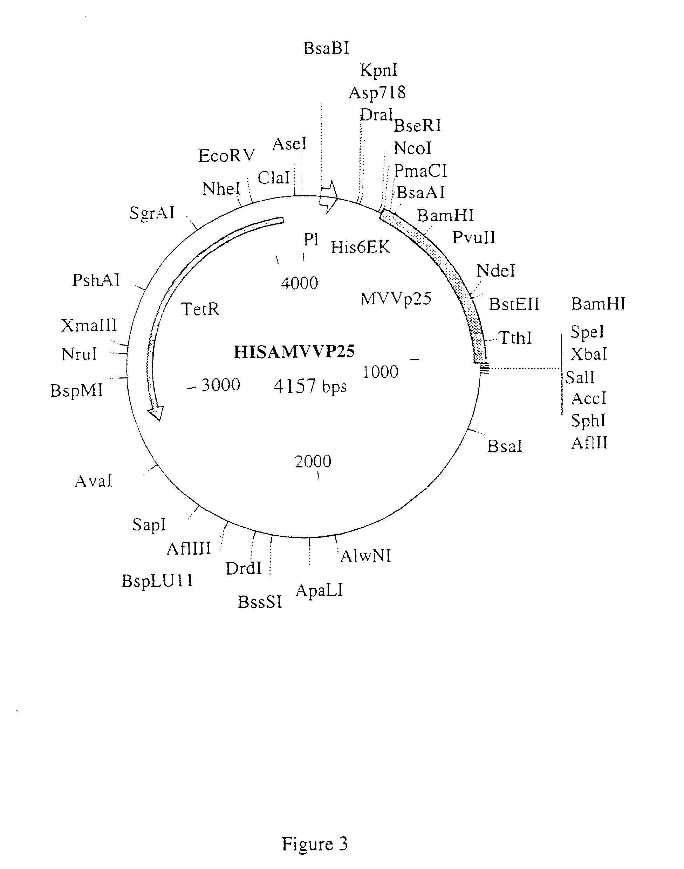

The expression vector pIGRHISA used in the present invention is represented in figure 3. The transcription of the heterologous gene is controlled by the leftward promoter of bacteriophage lambda. This promoter is controlled by a temperature sensitive repressor (Ci$^{ts}$) provided by a compatible plasmid present in the same cell. This system is tightly controlled and allows the cells to grow in repressed conditions (28°C) where no (or very limited) amounts of the heterologous protein is produced. Upon induction at higher temperature (37°C or 42°C) the protein of interest is produced during a limited period of time that can be optimized depending on the specific requirements for each protein to be produced. This system allows the production of proteins which are toxic to the host organism and the constitutive expression of which would be lethal for the host.

In the pIGRHISA vector, the initiation codon is followed by a codon for alanine immediately followed by six histidine codons and a cleavage site recognized by enterokinase. In this case, the protein produced carries at its N-terminal end the sequence M-A-H-H-H-H-H-H-V-D-D-D-K. The enterokinase cleavage site allows the removal of the hexahistidine sequence from the purified protein, should this prove necessary.

The gene encoding the p25 protein, obtained by PCR, was cloned into the pIGRHISA vector as a *Sma*I/*Xba*I digest. The vector was opened with *Nsi*I, blunted with Klenow polymerase and cut with *Xba*I. The *Sma*I/*Nsi*I fusion created in this way restores the reading frame between the His6 region from the vector and the MVV gene. This plasmid was termed pIGRHISAp25 (figure 3). The sequence of the recombinant p25 protein obtained from this construct is represented in figure 2 (SEQ ID NO 88). This recombinant protein will be designated HisMVVp25 protein.

*Induction of the heterologous expression*

Expression experiments are performed in *E. coli* strains carrying a compatible plasmid encoding a temperature sensitive mutant of the CI repressor gene. To induce expression of the genes cloned under control of the lambda promoter, a culture is started by inoculation from an overnight saturated culture at a 1/100 dilution. The culture is then grown at 28°C until an OD of about 0.2 (at 600 nm) and the temperature is shifted to 42°C. The culture is incubated further for several hours, the exact induction period being dependent on the individual protein. For large scale production of recombinant proteins, a 1 5l fermentor has been used. Induction conditions were essentially the same as described above. During the fermentation, the pH, oxygen pressure and temperature were monitored and adjusted. The growth curve is also registered. Upon termination of the fermentation, the cells are concentrated by microfiltration and centrifugation, and the cell pellet is stored at -70°C until further processing.

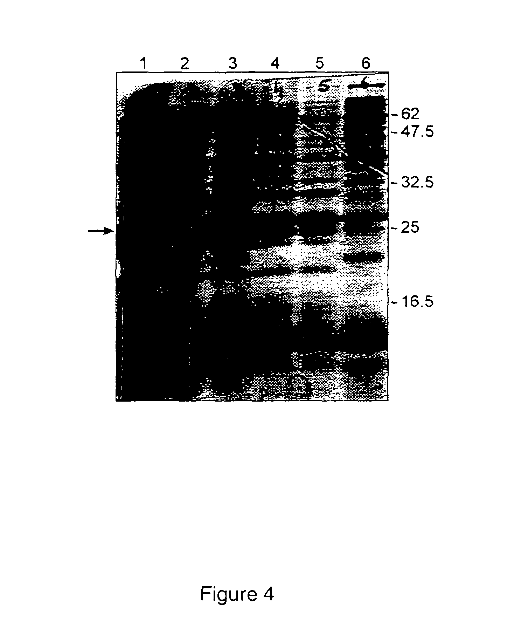

To evaluate the level of expression of the recombinant proteins, small scale inductions were performed and the cells were harvested at different time points after induction. Cells were lysed by boiling in SDS/mercapto-ethanol, and the lysate was analysed by SDS/PAGE and stained with coomassie blue. The expression level was analysed in three different *E. coli* strains (SG4044, MC1061 and UT5600). The highest expression level was obtained in SG4044 and this strain was used for large scale fermentation. Analysis of a fermentation run as a function of time is shown in figure 4. Moreover, figure 4 also shows the distribution of the protein between soluble and insoluble fraction (including the membrane fraction and inclusion bodies) after french press lysis of the cells. The MVV p25 recombinant protein is present in the soluble as well as in the insoluble fraction. Both fractions have been purified and used in serology.

*Purification of recombinant p25 protein*

The cell pellet obtained after fermentation was resuspended in lysis buffer (10 mM Tris pH 6.08, containing 150 mM KCl and 5 mM EDTA) (5 ml per gram of wet cells) and lysed by passing through the French press twice. Before lysis the suspension was supplemented with PMSF (1 mM) and epsilon-aminocaproic acid (25 mM) to inhibit proteases. The suspension was cleared by centrifugation and, since the MVV p25 protein is present in inclusion bodies as well as in the soluble fraction, both the cleared supernatant and the pellet were used for further processing.

The pellet fraction obtained after french press lysis was solubilized in buffer containing 6M guanidinium chloride (50 mM phosphate, 0.05% Triton-X-100, pH 7.2). The cleared solution obtained after centrifugation at 30.000 g was then applied to the preactivated Ni-IDA column (Pharmacia) equilibrated with the same buffer containing 6M guanidinium chloride and 20 mM imidazole. On a 12 ml IDA column, about 4 liter equivalent of fermentation broth was loaded. The column was then washed with the guanidinium buffer containing 0.05% Triton-X-100 until the absorption at 280 nm reached the basal level. The column was washed with the loading buffer containing 35 mM imidazole followed by 50 mM imidazole. The bound protein was then eluted with 200 mM imidazole. Fractions were analysed via SDS/PAGE and western blotting to evaluate the purity and the yield of the purification.

For the purification of the soluble fraction, the supernatant obtained after clearing of the french press lysate was supplemented with solid guanidinium chloride to 6M final concentration. Further processing was as described above.

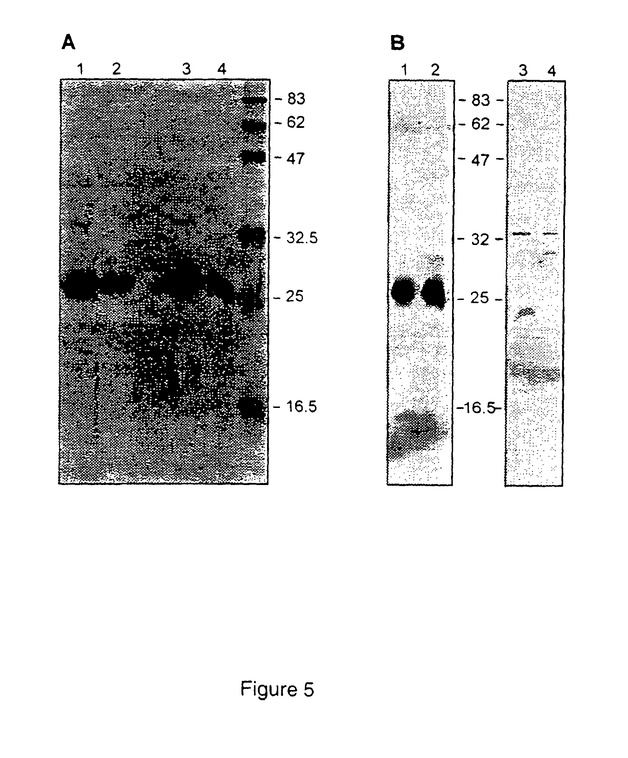

The protein fractions eluted from the IMAC column at 200 mM imidazole were pooled and analyzed on SDS/PAGE followed by immunoblotting (figure 5). The purity of the preparation was evaluated by probing the blot with serum raised to total *E. coli* protein. From this analysis the purity of both protein preparations was estimated to be higher than 95%. This was also confirmed by silver staining. The total yield of purified protein was about 10 mg HisMVVp25 protein per liter of fermentation broth.

The amount of purified recombinant protein was estimated by the micro BCA method (Pierce) using bovine serum albumin as a standard.

Fractions containing the recombinant HisMVVp25 protein were pooled and, before using them for serological analysis, they were dialysed against a buffer containing 6M urea (50 mM phosphate pH 7.2, 150 mM NaCl) to eliminate the guanidinium chloride and the imidazole, which have been shown to give high background with sheep sera (results not shown).

**<u>Example 2: Synthesis, biotinylation and cyclization of MVV TM-peptides</u>**

The peptides were synthesized on Tentagel S resin (Rapp Polymere GmbH,Germany) using a Rainin Symphony/Multiplex synthesizer with standard Fmoc-chemistry and HOBt/TBTU (TBTU:2-(1H-Benzotriazole- 1 -yl)-1,1,3,3-tetramethyluronium tetrafluoroborate; HOBt : N-Hydroxybenzotriazole) *in situ* activation. Standard double couplings were performed using a 4 fold excess of amino acids in the presence of equimolar amounts of HOBt and TBTU for 2 × 20 minutes . The Fmoc protecting group was removed by mild base treatment using 2%piperidine/2%DBU (1,8-Diazabicyclo[5.4.0]undec-7-ene) in DMF (Dimethylformamide).

Biotinylation was performed by dissolving biotin in 30% dimethylsulfoxide / 70% dimethylformamide with in situ activation. After completion of the peptide-resin complex the peptide is cleaved off the resin by incubating for 2,5 h with 90% trifluoroacetic acid/ 5% thioanisole/3% ethanedithiol/2% anisole. The peptide is precipitated from the cleaving mixture using t-butylmethylether. After centrifugation the pellet is washed 3 times with t-butylmethylether and dried overnight in vacuum. The purity of the crude peptide is checked on RP-HPLC.

The following TM peptides were synthesized:

TM1: B-GGQELDCWHYQHYCVTSTKS (SEQ ID NO 85)

TM2: B-GGRYRDNCTWQQWEEEIE (SEQ ID NO 86)

## TM3: B-GGQRDAQRIPDVWTALQG (SEQ ID NO 87)

Oxidation (cyclization) of the TM1 peptide was carried out according to the following procedure: 48.4 mg of TM1 was dissolved in 242 ml 20% ACN (Acetonitrile)/0.1% TFA (sonication). 20 µl samples were taken as reference starting material on analytical scale. $K_3[Fe(CN)_6]$ was added at a final concentration of 0.24 mM. The pH was increased to pH11 with 3M NaOH and the solution was stirred for 150 min at room temperature and covered from light. Finally, the pH was lowered again to pH 1.5 using TFA. Storage at -20°C.

After oxidation of the TM1 peptide, the oxidized cyclic peptide (TM1c) was separated from the remaining non-oxidized fraction by HPLC chromatography. Briefly, the sample was loaded on the HPLC column (Nucleosil 10µ (C18) column distributed by Alltech) in 0.1% TFA. Elution was performed with a gradual increase of ACN, using a solution of 80% ACN in 0.1% TFA. The TM1c peptide eluted at 28-30% ACN, while the linear TM1 peptide eluted as a separate peak at 31-32 % ACN.

## Example 3. Serological evaluation of the MVV TM-peptides and p25 antigen

*Serum samples*

The MVV peptides and proteins of the invention have been tested against a collection of sera from MVV infected and non-infected sheep, in order to assess their usefulness as immunodiagnostic reagents. Sheep were mainly of the Rasa meat breed. All animals were 6 months of age or older.

The sera from infected animals were characterized by the classical AGIDT (Agar Gel Immuno Diffusion Test), using a commercial kit (Maeditec-1000, Central Veterinary Laboratory, U.K.). In this kit, MVV complete viral particles serve as antigens and a reference serum against gp135 and p25 is provided as antibody control. Interpretation of the results was carried out according to the manufacturer's instructions. Sera were classified as positive for gp135 (gp 1 35 +), weakly positive for gp 135 (gp 1 35 +/-), positive for p25, doubtful (indeterminate) and negative. Sera from Icelandic sheep free of MVV were used as certified seronegative controls. All sera were stored at -20°C.

*ELISA procedure*

Recombinant p25 antigen and the TM peptides were evaluated in ELISA for their sensitivity and specificity to detect sera from MVV infected sheep.

Coating conditions (pH of coating buffer, optimal antigen concentration...) were determined and optimized in order to obtain the most favorable signal to noise (S/N) ratio. The antigens are diluted from a stock solution into the coating buffer to obtain the desired antigen concentration. The dilution rate is at least 1 /100 to minimize the possible interference from ureum present in the protein stock solution. The biotinylated peptides were coated either directly (i. e. without streptavidin complexation), or after complex formation with streptavidin. It should be understood that the direct coating of biotinylated peptides can be considered to have an equivalent effect as the coating of non-biotinylated peptides. The complex formation with streptavidin can occur either in solution (i.e. before coating) or on plates which have been previously coated ("sensitized") with streptavidin (3 µg/ml in carbonate buffer). In the following examples, the complexation of TM peptides with streptavidin occurs prior to coating ( = complex coating).

The coating conditions used (unless stated differently) are shown below in <u>table 3</u>:

Table 3:

| ELISA coating conditions for MVV antigens (peptides; proteins) | |
|---|---|
| **antigen** | **coating conditions (all at 100 µl/well for 1h at 37°C)** |
| p25 | 5 µg/ml in carbonate buffer pH9.6 |
| TM peptides Direct coating | 1 µg/ml TM peptide in carbonate buffer pH9.6 |
| TM peptides Complex coating | 1 µg/ml complex * [strept/TM] in carbonate buffer pH9.6 |
| combi complex | 1 µg/ml p25 and 3 µg/ml complex * [strept/TM] in carbonate buffer pH9.6 |

\*: complex:       9 µl streptavidine (5 mg/ml)
            12 µl TM peptide (1 mg/ml)
        129 µl carbonate buffer
incubate the mix for 1h at 37°C

After coating, the plates were saturated with a casein solution (0.1 % casein in PBS) plus 0.25 M glycine ("blocking buffer"). The wells were incubated with 250 $\mu$l of blocking buffer for 1h at 37 °C, after which they were washed 3 times with a solution of PBS-Tween 20 ("washing buffer"). Serum samples, diluted 1/500 in "sample diluent" (blocking buffer + 2.86 g/l Triton X-705), were incubated at 100 $\mu$l/well for 1h at 37°C. After extensive (5x) washing with washing buffer, detection of the bound sheep antibodies was performed with a peroxidase conjugated immunoglobuline rabbit anti-sheep (Prosan), diluted at 1/10.000 in blocking buffer, which was left in the wells for 1 h at 37 °C. After another extensive washing round (5x), 100 $\mu$l of chromogen TMB (tetramethylbenzidin) was added to the wells (1/100 dilution in substrate buffer (0.06M $Na_2HPO_4.2H_2O$; 0.04M citric acid; 0.006% $H_2O_2$). After 15-30 min, color development was stopped by addition of 100 $\mu$l 1N $H_2SO_4$ and quantified by measurement of optical density (OD) values at 450 nm - 595 nm. ELISA results may be expressed as OD values, or as S/N ratio (Signal/Noise), with N being determined in each separate experiment with a set of 15 certified negative icelandic sera (N = 2x (mean of negative OD values + 3SD)). ELISA results are considered positive when the S/N ratio is over 1.

*Influence of streptavidin complex formation on the seroreactivity of TM1*

The biotinylated TM1 peptide (SEQ ID NO 85) was coated either directly on the microtiter plates, or after complex formation to streptavidin, as described above. As illustrated in figure 6 and table 4, the OD values drastically increased upon presentation of the peptide via biotin-streptavidin interaction. Since direct coating of the biotinylated TM1 peptide can be considered equivalent to the direct coating of the non-biotinylated TM1 peptide, it can be said that biotinylation of the TM1 peptide and subsequent presentation of the peptide on a solid phase via streptavidin interaction are indispensable in order to obtain a good reactivity of the peptide with the sheep sera. More bluntly said, if the peptide represented by SEQ ID NO 1 is coated as such on the surface of an immunoassay solid phase, it is completely useless (see figure 6a). Upon biotinylation of the peptide (such as in SEQ ID NO 85) and subsequent presentation of the peptide via biotin-streptavidin interaction it becomes highly reactive with sera from MVV infected sheep (see figure 6b). Although it is known that presentation of the peptide via a streptavidin/biotin complex may have a positive effect on the seroreactivity and/or binding efficiency of some peptides, the difference in reactivity observed between figure 6a and 6b may be considered as highly surprising. As shown in Table 4, the observed increase in reactivity often approached or even exceeded a factor of 100.

**Table 4:** OD values corresponding to the ELISA represented in Figure 6. Increase factor = factor of increase in reactivity when the biotinylated TM peptide is coated via streptavidin instead of direct coating on the microtiter plate.

Direct coating

| Serum | TM1 |
|---|---|
| N-1/4 | 0.0105 |
| N-1/8 | 0.0065 |
| N-1/9 | 0.007 |
| N-1/10 | 0.053 |
| N-1/14 | 0.005 |
| N-1/19 | 0.0055 |
| N-1/20 | 0.023 |
| N-1/24 | 0.018 |
| N-3/2 | 0.016 |
| N-3/3 | 0.008 |
| N-3/4 | 0.01 |
| N-3/10 | 0.0325 |
| N-3/14 | 0.009 |
| N-3/15 | 0.0175 |
| N-3/19 | 0.0105 |
| N-3/23 | 0.0095 |
| N-3/25 | 0.0055 |
| N-26/9 | 0.006 |
| N-26/19 | 0.007 |
| N-1/3 | 0.013 |
| N-1/13 | 0.004 |
| N-3/8 | 0.018 |
| N-3/9 | 0.007 |
| N-3/21 | 0.013 |
| N-24/15 | 0.0075 |
| N-26/1 | 0.01 |
| N-27/16 | 0.0455 |
| N-3/1 | 0.007 |
| N-3/6 | 0.011 |
| N-3/18 | 0.006 |

Streptavidin coating

| Serum | TM1 | -TM1 | increase factor |
|---|---|---|---|
| N-1/4 | 0.843 | 0.042 | 76 |
| N-1/8 | 0.026 | 0.022 | |
| N-1/9 | 0.402 | 0.028 | 53 |
| N-1/10 | 0.187 | 0.01 | 3 |
| N-1/14 | 0.352 | 0.017 | 67 |
| N-1/19 | 0.516 | | 94 |
| N-1/20 | 0.172 | 0.037 | 6 |
| N-1/24 | 1.789 | | 99 |
| N-3/2 | 0.743 | 0.06 | 43 |
| N-3/3 | 0.836 | 0.012 | 103 |
| N-3/4 | 1.206 | 0.022 | 118 |
| N-3/10 | 1.24 | 0.033 | 37 |
| N-3/14 | 0.205 | 0.012 | 21 |
| N-3/15 | 1.612 | 0.031 | 90 |
| N-3/19 | 0.654 | 0.024 | 60 |
| N-3/23 | 1.297 | 0.02 | 134 |
| N-3/25 | 0.314 | 0.013 | 55 |
| N-26/9 | 1.525 | 0.018 | 251 |
| N-26/19 | 0.315 | 0.016 | 43 |
| N-1/3 | 1.061 | 0.014 | 81 |
| N-1/13 | 0.366 | 0.042 | 81 |
| N-3/8 | 0.134 | 0.069 | 4 |
| N-3/9 | 1.042 | 0.027 | 145 |
| N-3/21 | 0.1 | 0.043 | 4 |
| N-24/15 | 1.071 | 0.015 | 141 |
| N-26/1 | 0.153 | 0.016 | 14 |
| N-27/16 | 0.068 | 0.075 | |
| N-3/1 | 0.036 | 0.024 | 2 |
| N-3/6 | 0.857 | 0.055 | 73 |
| N-3/18 | 1.21 | 0.014 | 199 |

| | |
|---|---|
| N-1/2 | 0.008 |
| N-1/15 | 0.02 |
| N-1/17 | 0.0065 |
| N-24/1 | 0.005 |
| N-24/2 | 0.0055 |
| N-24/8 | 0.033 |
| N-24/19 | 0.0105 |
| N-26/17 | 0.007 |
| *3148 | 0.006 |
| *3215 | 0.0045 |
| *3265 | 0.0045 |
| *3396 | 0.006 |
| *3398 | 0.004 |
| *3408 | 0.0045 |
| *3508 | 0.017 |
| *3518 | 0.004 |
| *3600 | 0.004 |
| *3628 | 0.004 |
| c.o. | 0.018 |

| | | | |
|---|---|---|---|
| N-1/2 | 1.292 | 0.044 | 156 |
| N-1/15 | 0.28 | 0.059 | 11 |
| N-1/17 | 0.021 | 0.02 | |
| N-24/1 | 0.326 | 0.015 | 62 |
| N-24/2 | 0.091 | 0.021 | 13 |
| N-24/8 | 0.591 | 0.111 | 15 |
| N-24/19 | 0.12 | 0.026 | 9 |
| N-26/17 | 0.521 | 0.042 | 68 |
| *3148 | 0.027 | 0.013 | |
| *3215 | 0.025 | 0.017 | |
| *3265 | 0.018 | 0.019 | |
| *3396 | 0.016 | 0.017 | |
| *3398 | 0.028 | 0.021 | |
| *3408 | 0.02 | 0.019 | |
| *3508 | 0.053 | 0.052 | |
| *3518 | 0.021 | 0.019 | |
| *3600 | 0.02 | 0.019 | |
| *3628 | 0.025 | 0.024 | |
| c.o. | 0.057 | | |

*Comparative seroreactivity of peptides TM1, TM2 and TM3 in ELISA*

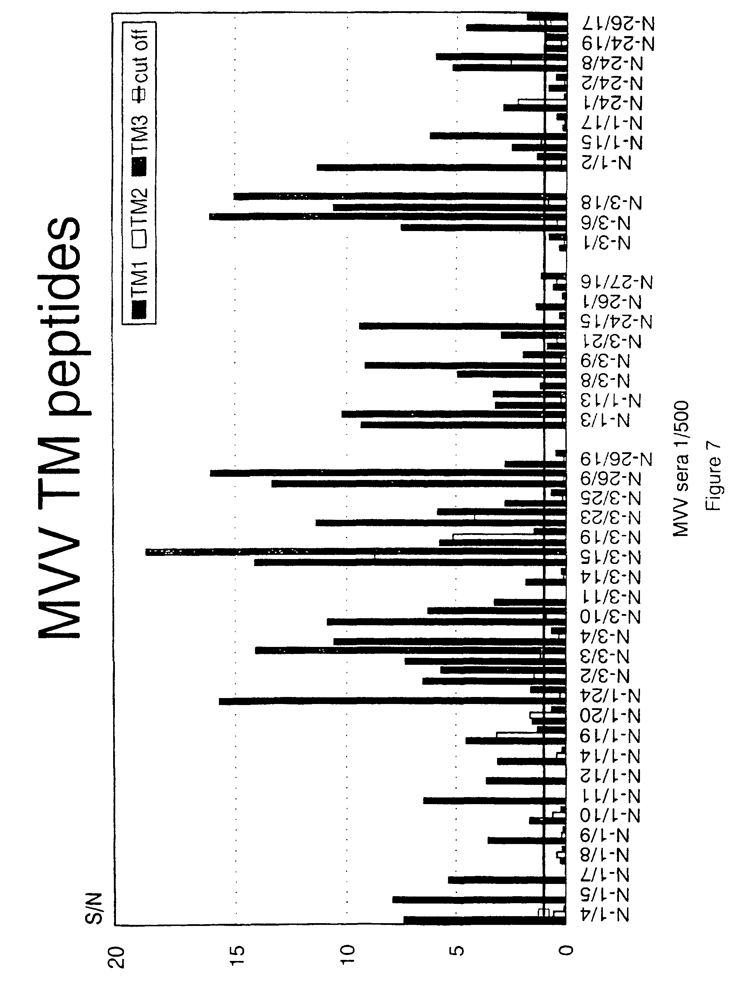

The three biotinylated TM peptides TM1, TM2 and TM3 (SEQ ID NO 85, 86 resp. 87) were complexed to streptavidin, and subsequently coated on microtiter plates for serological testing. The same set of sera as presented in figure 6 were used. Figure 7 shows the clear superior reactivity of the TM1 peptide as compared to TM2 and TM3, which were only marginally reactive with MVV AGIDT positive sera. On the total group of sera tested in figure 7, the overall sensitivity obtained with TM1 as detecting reagent is 84%, while an ELISA based on TM2 or TM3 only reaches a sensitivity level of 19% and 42% respectively.

*Seroreactivity of the p25 antigen*

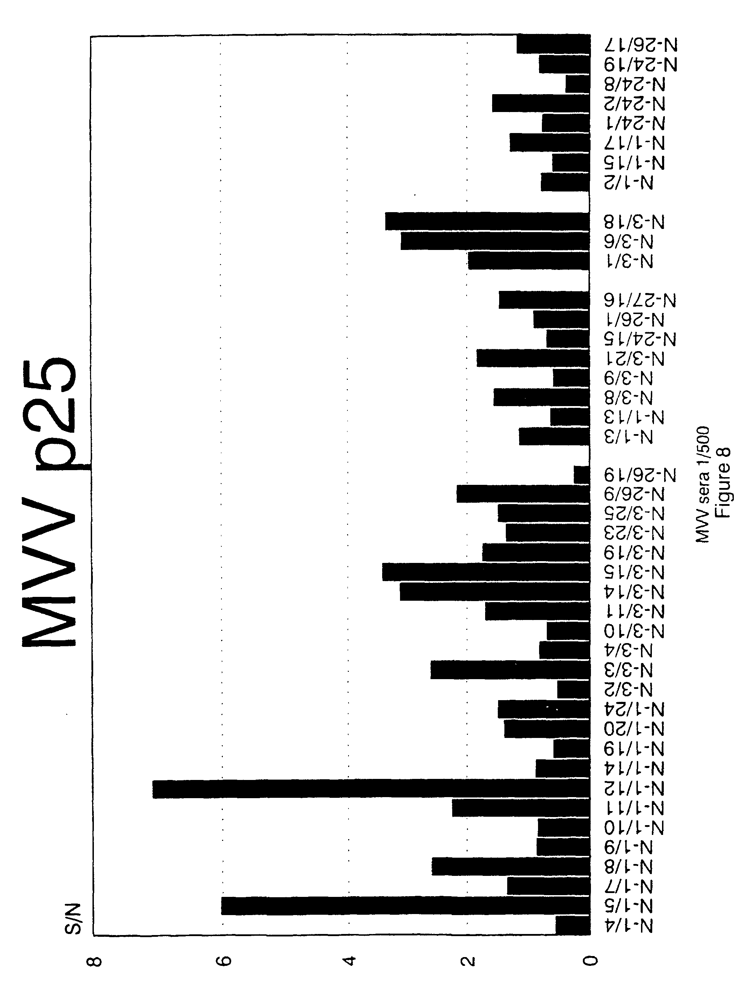

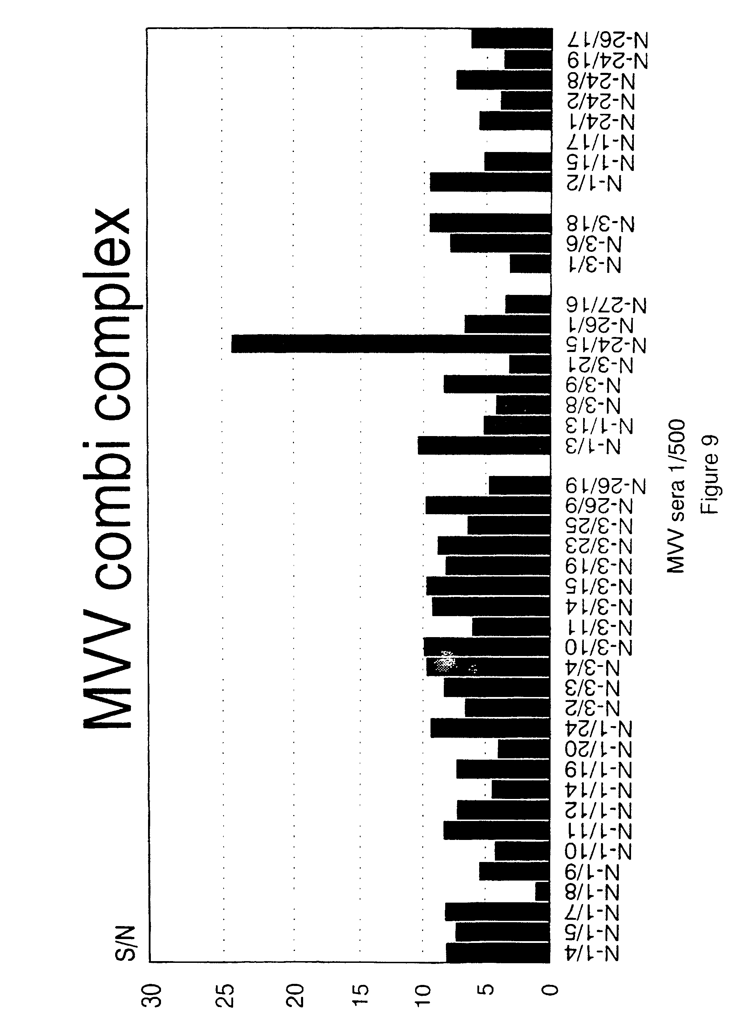

The same set of sera was used to evaluate the serological reactivity of the recombinant p25 antigen, under the conditions as described above. Results are shown in figure 8. Some sera, which were missed out with the peptide TM1, are now detected with the p25 antigen (such as serum nr. N-1/8, N-3/21 ). On the other hand, the overall sensitivity of the p25 antigen is lower (58%) than the sensitivity obtained with the peptide TM1 alone (84%). Moreover, S/N values obtained with the p25 antigen were usually lower than the values obtained using TM1 peptide as an antigen.

In conclusion, the high sensitivity obtained with the TM1 peptide, superior to the sensitivity obtained with the recombinant p25 can be considered as quite exceptional, and proves the truly immunodominant character of the epitope represented by TM1. A limited number of sera seems to be unreactive with the peptide but reacts with the recombinant p25. Therefore, a combined ELISA using both antigens together on the solid phase was designed.

*ELISA based on a combination of the TM1 peptide with p25: "Combi-ELISA"*

A combination of the p25 antigen with the biotinylated TM1 peptide, complexed to streptavidin, was tested again with the same set of sera as before. Coating conditions needed to be optimised first. When both antigens, p25 and [strept/TM1] complex were applied in the concentrations as used for the separate coating, the signal obtained with several sera was considerably lower than when the antigens were used separately. This was particularly the case for sera giving high signals with the TM1 peptide alone, but negative with p25. This observation indicated that the recombinant p25 was having an adverse effect on the TM1 reaction. Therefore, different ratio of p25 and TM1 peptide were tested for coating and the results showed that lowering the coating concentration of the p25 from 5μg/ml to 1μg/ml improved the results considerably when both antigens were coated together. Therefore, the conditions finally adopted for the combined coating are p25 at 1μg/ml and complex [strept/TM1] at 3μg/ml as shown in table 3. Table 5 shows that, using this combination of antigens, an exceptional high sensitivity of (> 98%) was obtained.

A number of additional serum samples from different origin (Scotland, Spain, Italy) have been tested in the Combi-ELISA. The overall evaluation is summarized in the Table 5 below.

Table 5.

| Summary of serological results obtained with Combi-ELISA compared to AGIDT (total number of sera tested: 2336) | | | |
|---|---|---|---|
| | **AGIDT +** | **AGIDT -** | **AGIDT + /-** |
| **ELISA +** | 620 | 46 | 90 |
| **ELISA -** | 10 | 1564 | 6 |
| | **Sensitivity 98.4%** | **Specificity 97.1 %** | |

The overall concordance between AGIDT and ELISA is good as far as the sensitivity is concerned (98.4%). This was to be expected since it is known that ELISA is more sensitive than AGIDT. On the other hand, this higher sensitivity has as a consequence that more AGIDT indetermined or AGIDT negative samples are found positive in ELISA. The relative insensitivity of the AGIDT test makes it unsuitable as a gold standard, hence, the lower specificity score (96.9%) of the ELISA versus AGIDT has to be considered with caution. Samples resulting in discrepant results, were analysed further using alternative serological approaches (such as LIA, or western blotting using viral lysate as an antigen). Taking into account the results from the alternative serology, the figures from Table 5 are adapted as shown in Table 6 (leaving out the indeterminate samples). This table illustrates that taking into account the results from alternative serological tests has a considerable effect on the specificity score of the assay.

Table 6.

| Summary of serological results obtained with Combi-ELISA considering the alternative serological testings | | |
|---|---|---|
| | **Truly +** | **Truly -** |
| **ELISA +** | 655 | 11 |
| **ELISA -** | 4 | 1570 |
| | **Sensitivity 99.4%** | **Specificity 99.3%** |

*Influence of cyclization of the TM1 peptide*

It was noted during serological evaluation of the TM1 peptide that the reactivity of freshly dissolved TM1 was suboptimal, and reactivity improved upon storage of the solution (see table 7). This phenomenon could indicate that disulfide bridge formation was taking place during storage.

Table 7:

| S/N ratio obtained with a combi-ELISA wherein either a new (= freshly dissolved) (N) or old (= stored) (O) solution of TM1 is used as coating antigen in combination with p25. Sera 6 to 118 originate from Schotland (AGIDT+ sera). The other sera (N) originate from Zaragoza (Spain). | | |
|---|---|---|
| **serum nr.** | **O-TM1** | **N-TM1** |
| 6 | 12.44 | 7.24 |
| 11 | 12.11 | 6.5 |
| 25 | 8.48 | 5.41 |

Table 7: (continued)

| S/N ratio obtained with a combi-ELISA wherein either a new (= freshly dissolved) (N) or old (= stored) (O) solution of TM1 is used as coating antigen in combination with p25. Sera 6 to 118 originate from Schotland (AGIDT+ sera). The other sera (N) originate from Zaragoza (Spain). | | |
|---|---|---|
| serum nr. | O-TM1 | N-TM1 |
| 45 | 3.98 | 2.18 |
| 65 | 7.24 | 3.51 |
| 71 | 11.84 | 6.33 |
| 75 | 11.92 | 6.33 |
| 78 | 20.26 | 15.44 |
| 101 | 12.37 | 6.64 |
| 103 | 11.61 | 7.33 |
| 115 | 5.74 | 3.79 |
| 118 | 13.42 | 8.09 |
| N-1/4 | 13.82 | 7.95 |
| N-1/5 | 19.48 | 11.9 |
| N-1/7 | 14.26 | 10.74 |
| N-1/8 | 2.03 | 1.5 |

Chemical oxidation of the TM1 peptide to form intramolecular disulfide bridges was performed as described above. This treatment produced a peptide (TM1c) of good initial reactivity and stability in solution. It was therefore used as a preferred reagent for the development of serological assays over TM1.

*Alternative serological assays*

As an alternative system, the antigens were also applied on LIA (Line Immuno Assay) strips. In this system, the purified antigens or peptide complexes were applied in parallel lines onto nylon membranes which were then cut perpendicular to the antigen lines in order to obtain small strips (3-5 mm width). Peptides were applied as complexes with streptavidin. These strips were incubated with the diluted serum and further developed similar to a western blot. Reactivity of the antigen or peptide with the serum is revealed as a coloured line on the respective positions.
Results obtained indicated that LIA may be useful for further testing of problematic (doubtful) sera. Indeed, in some cases, ELISA negative samples which are AGIDT positive were reactive with the p25 protein on LIA. This indicates that the LIA may be useful for confirmation purposes in ambiguous cases.

*Testing of goat sera*

A limited number of goat sera (212) were tested using the combi-ELISA under identical conditions as described for the sheep sera. The detecting antibody (peroxidase conjugated immunoglobuline rabbit anti-sheep (Prosan)) shows sufficient cross reactivity with goat antibodies, in order to be used as a detecting agent in the combi-ELISA for CAEV detection in goat. Table 8 below shows the results obtained. It is clear that the Combi-ELISA, as described above, based on the MVV TM1 peptide (SEQ ID NO 85) and the MVV p25 recombinant protein, is perfectly suitable also for the detection of anti-CAEV antibodies in goats, besides its demonstrated usefulness for detection of MVV infection in sheep.

**EP 1 073 676 B1**

Table 8.

| Summary of serological results obtained on goat sera with Combi-ELISA compared to AGIDT (total number of sera tested:212) | | |
|---|---|---|
| | **AGIDT +** | **AGIDT -** |
| **ELISA +** | 142 | 14 |
| **ELISA -** | 0 | 56 |

REFERENCES

Bertoni, G., Zahno, M.L., Zanoni, R. et al. (1994) Antibody reactivity to the immunodominant epitopes of the caprine arthritis encephalitis virus gp38 transmembrane protein associates with the development of arthritis. J. Of Virology 68, 11, 7139-7147.

Bodansky M. 1993. Principles of Peptide synthesis, 2nd Edition. Springer Laboratory by Springer-Verlag.

Boshoff, C.H., Dungu, B., Williams, R. et al. (1997) Detection of Maedi-Visna virus antibodies using a single fusion transmembrane-core p25 recombinant protein ELISA and a modified receiver-operating characteristic analysis to determine cut-off values. J. Virol. Meth. 63, 47-56.

Bulgin, M.S. (1990) Ovine progressive pneumonia, caprine arthritis-encephalitis, and related lentiviral diseases of sheep and goats. Vet. Clin. N. Am. Food Anim. Pract. 6(3), 691-703.

Campbell, J.R., Menzies, P., Waltner-Toews, D. et al. (1994) The seroprevalence of maedi-visna in Ontario sheep flocks and its relationship to flock demographic and management practices. Can. Vet. J. 35, 39-44.

Coackley, W and Smith, V.W. (1984) Preparation and evaluation of antigens used in serological tests for caprine syncytial retrovirus antibody in sheep and goat sera. Vet. Microbiol. 9, 581-586.

Cutlip, R.C. and Jackson, T.A. (1977) Immunodiffusion test for ovine progressive pneumonia. Am. J. Vet. Res. 38, 1081-1084.

Cutlip, R.C. and Laird, G.A. (1976) Isolation and characterization of a virus associated with progressive pneumonia (maedi) of sheep. Am. J. Vet. Res. 37, 1377-1382.

Hoff-Jörgensen, R. (1990) Diagnostic methods. In: G. Petursson and R. Hoff-Jorgensen (Editors), Maedi-Visna and related diseases. Kluwer, Boston/Dordrecht/London, p. 75-82.

Houwers, D.J. and Schaake, J. (1987) An improved ELISA for the detection of antibodies to ovine and caprine lentiviruses, employing monoclonal antibodies in a one-step assay. J. Immunol. Methods, 98, 151-154.

Houwers, D.J., Gielkins, S.L.J. and Schaake (1982) An indirect enzyme linked immunosorbent assay (ELISA) for the detection of antibodies to maedi visna virus. Vet. Microbiol. 7, 209-219.

Houwers, D.J. and Schaake, J. (1987) An improved ELISA for the detection of antibodies to ovine and caprine lentiviruses, employing monoclonal antibodies in a one step assay. J. Immunol. Methods 98, 151-154.

Keen, J., Kwang, J. and Rosati, S. (1995) Comparison of ovine lentivirus detection by conventional and recombinant serological methods. Vet. Immunol. and Immunopath. 47, 295-309.

Kwang, J. and Cutlip, R.C. (1992a) Analysis of antibody response to ovine lentivirus by using viral gene products expressed in a prokaryotic system. Biochem. Biophys. Res. Comm. 188(1), 20-27.

Kwang, J., Cutlip, R. (1992b) Detection of antibodies to ovine lentivirus using a recombinant antigen derived from the env gene. Biochem Biophys Res Commun 183, 1040.

Kwang, J. and Torres, J. (1994) Oligopeptide based enzyme immunoassay for ovine lentivirus antibody detection. J. Clin. Microbiol. 32, 7, 1813-1815. Kwang, J., Kim, H.S., Rosati S. et al. (1995) Characterization of ovine lentivirus envelope glycoprotein expressed in Eschericia coli and baculovirus systems. Vet Immunol Immunopathol 45, 185.

Maniatis T., Fritsch E.F. and Sambrook J. (1982) Molecular cloning. A laboratory manual. Cold Spring Harbor Labs. NY

McCurdy S.N. (1989) Pept. Res. 2, 147.

Myer, M.S., Huchzermeyer, H.F., York D.F. et al. (1988) The possible involvement of immunosuppression caused by a lentivirus in the aetiology of Jaagsiekte and Pasteurellosis in sheep. Onderstepoort J. Vet. Res. 55(3), 127-133.

Pancino, G., Ellerbrok, M., Sitbon, M. et al. (1994) Conserved framework of envelope glycoproteins among lentiviruses. Curr. Top. Microbiol. Immunol. 188, 77-105.

Pohl, M. et al. (1993) Int. J. Peptide Protein Res. 41, 362.

Power, C., Richardson, S., Briscoe, M. et al. (1995) Evaluation of two recombinant maedi-visna virus proteins for use in an enzyme linked immunosorbent assay for detection of antibodies to ovine lentiviruses. Clin. Diagn. Lab. Immunol. 2, 631.

Sargan, D.R., Bennet, I.D., Cousens, C. et al. (1991) Nucleotide sequence of EV 1, a British isolate of maedi-visna virus. J. Gen. Virol. 72, 1893-1903.

Simard, C.L. and Briscoe, M.R. (1990) An enzyme linked immunosorbent assay for detection of antibodies to maedi-visna virus in sheep II. Comparison to conventional agar gel immunodiffusion test. Can. J. Vet. Res. 54, 4451-4456.

Zanoni, R., Nauta, I., Pauli, U. et al. 1991. Expression in *E. coli* and sequencing of the coding region for the capsid protein of Dutch Maedi Visna virus strain ZZV 1050: application of recombinant protein in ELISA for the detection of caprine and ovine lentiviruses. J. Clin. Microb. 29, 7, 1290-1294.

Zwahlen, R., Aeschbacher, M., Balcker, T. Et al. 1983. Lentivirusinfektionen bei Ziegen mit Carpitis und interstitieller Mastitis. Schweiz. Arch. Tierheilk. 125: 281-299.

## Claims

1.  A biotinylated peptide having the following amino acid sequence:

$$\text{B-GGQELDCWHYQHYCVTSTKS (SEQ ID N0 85)}$$

2.  A biotinylated peptide according to claim 1, whereby the two cysteine (C) residues present in the amino acid sequence are covalently linked to form an intramolecular cysteine bridge.

3.  A biotinylated peptide according to any of claims 1 to 2, whereby the biotinyl moiety is complexed to a streptavidin or avidin molecule.

4.  A peptide composition comprising a biotinylated peptide according to any of claims 1 to 3, in combination with any other MVV peptide or recombinant protein.

5.  A peptide composition according to claim 4 comprising a biotinylated peptide according to any of claims 1 to 3 in combination with a recombinant MVV *gag* (p25) polypeptide, or a fragment thereof, more specifically, in combination with the recombinant MVV p25 protein as represented by SEQ ID NO 88 (figure 2).

6.  Use of a biotinylated peptide or peptide compositions according to any of claims 1 to 3 or claims 4 to 5 for the immunodetection of MVV or CAEV infection.

7.  Use of a biotinylated peptide or peptide compositions according to any of claims 1 to 5 in a kit for the immunodetection of MVV or CAEV infection.

8.  A solid support upon which a biotinylated peptide according to any of claims 1 to 3, or a peptide composition according to any of claims 4 to 5, has been immobilized.

9.  A method for the immunodetection of MVV infection or CAEV infection in mammals, comprising the steps of:

    -   contacting a biological sample from said mammal with a biotinylated peptide according to any of claims I to 3, or with a peptide composition according to any of claims 4 to 5, under conditions allowing the formation of an immunological complex, and
    -   detecting the presence of an immunological complex formed between said biotinylated peptide or said peptide composition and the anti-MVV or anti-CAEV antibodies which may be present in the sample.

10. An immunodiagnostic kit for the detection of MVV infection or CAEV infection in mammals, said kit comprising a biotinylated peptide according to any of claims 1 to 3, or a peptide composition according to any of claims 4 to 5.

**Patentansprüche**

1.  Biotinyliertes Peptid mit der folgenden Aminosäuresequenz:

    B–GGQELDCWHYQHYCVTSTKS (SEQ ID NO 85)

2.  Biotinyliertes Peptid nach Anspruch 1, wobei die beiden in der Aminosäuresequenz vorhandenen Cysteinreste (C)unter Bildung einer intramolekularen Cysteinbrücke kovalent verknüpft sind.

3.  Biotinyliertes Peptid nach Anspruch 1 oder 2, wobei die Biotinylgruppierung in einem Komplex mit einem Streptavidin- oder Avidinmolekül vorliegt.

4.  Peptidzusammensetzung, umfassend ein biotinyliertes Peptid nach einem der Ansprüche 1 bis 3, kombiniert mit einem beliebigen anderen MVV-Peptid oder rekombinanten Protein.

5.  Peptidzusammensetzung nach Anspruch 4, umfassend ein biotinyliertes Peptid nach einem der Ansprüche 1 bis 3, kombiniert mit einem rekombinanten MVV-*gag*(p25)-Polypeptid oder einem Fragment davon, insbesondere kombiniert mit dem rekombinanten Protein MVV-p25, wie es durch SEQ ID NO 88 (Figur 2) dargestellt ist.

6.  Verwendung eines biotinylierten Peptids oder von biotinylierten Peptidzusammensetzungen nach einem der Ansprüche 1 bis 3 bzw. Ansprüchen 4 und 5 zum immunologischen Nachweis einer Infektion mit MVV oder CAEV.

7.  Verwendung eines biotinylierten Peptids oder von biotinylierten Peptidzusammensetzungen nach einem der Ansprüche 1 bis 5 in einem Kit zum immunologischen Nachweis einer Infektion mit MVV oder CAEV.

8.  Fester Träger, an dem ein biotinyliertes Peptid nach einem der Ansprüche 1 bis 3 oder eine Peptidzusammensetzung nach Anspruch 4 oder 5 immobilisiert ist.

9.  Verfahren zum immunologischen Nachweis einer MVV- oder CAEV-Infektion in Säugern, bei dem man:

    -   eine biologische Probe aus dem Säuger mit einem biotinylierten Peptid nach einem der Ansprüche 1 bis 3 oder einer Peptidzusammensetzung nach Anspruch 4 oder 5 unter Bedingungen, die die Bildung eines immunologischen Komplexes gestatten, in Kontakt bringt und
    -   das Vorhandensein eines zwischen dem biotinylierten Peptid oder der Peptidzusammensetzung und den gegebenenfalls in der Probe vorhandenen Anti-MVV- bzw. Anti-CAEV-Antikörpern ausgebildeten immunologischen Komplexes nachweist.

10. Immundiagnostischer Kit zum Nachweis einer MVV- oder CAEV-Infektion in Säugern, wobei der Kit ein biotinyliertes Peptid nach einem der Ansprüche 1 bis 3 oder eine Peptidzusammensetzung nach Anspruch 4 oder 5 umfaßt.

**Revendications**

1.  Peptide biotinylé ayant la séquence d'acides aminés suivante :

    B–GGQELDCWHYQHYCVTSTKS (SEQ ID n° 85)

2.  Peptide biotinylé selon la revendication 1, moyennant quoi les deux résidus de cystéine (C), présents dans la séquence d'acides aminés, sont liés de manière covalente pour former un pont intramoléculaire de cystéines.

3.  Peptide biotinylé selon l'une quelconque des revendications 1 à 2, moyennant quoi la fraction biotinyl est complexée à une molécule de streptavidine ou d'avidine.

4.  Composition peptidique comprenant un peptide biotinylé, selon l'une quelconque des revendications 1 à 3, en combinaison avec n'importe quel autre peptide ou protéine recombinante de MVV.

**EP 1 073 676 B1**

5. Composition peptidique, selon la revendication 4, comprenant un peptide biotinylé, selon l'une quelconque des revendications 1 à 3, en combinaison avec un polypeptide *gag* (p25) de MVV ou un fragment de celui-ci, plus spécifiquement en combinaison avec la protéine p25 de MVV recombinante, comme cela est représenté par la SEQ ID n° 88 (figure 2).

6. Utilisation d'un peptide biotinylé ou de compositions peptidiques, selon l'une quelconque des revendications 1 à 3 ou des revendications 4 à 5, pour l'immunodétection d'une infection à MVV ou à CAEV.

7. Utilisation d'un peptide biotinylé ou de compositions peptidiques, selon l'une quelconque des revendications 1 à 5, dans un kit pour l'immunodétection d'une infection à MVV ou à CAEV.

8. Support solide sur lequel un peptide biotinylé, selon l'une quelconque des revendications 1 à 3, ou une composition peptidique, selon l'une quelconque des revendications 4 à 5, a été immobilisé.

9. Procédé pour l'immunodétection d'une infection à MVV ou d'une infection à CAEV chez des mammifères, comprenant les étapes consistant à :

   - mettre en contact un échantillon biologique provenant dudit mammifère avec un peptide biotinylé, selon l'une quelconque des revendications 1 à 3, ou avec une composition peptidique, selon l'une quelconque des revendications 4 à 5, dans des conditions qui permettent la formation d'un complexe immunologique ; et
   - détecter la présence d'un complexe immunologique s'étant formé entre ledit peptide biotinylé ou ladite composition peptidique et les anticorps anti-MVV ou anti-CAEV qui peuvent être présents dans l'échantillon.

10. Kit d'immunodiagnostic pour la détection d'une infection à MVV ou d'une infection à CAEV chez des mammifères, ledit kit comprenant un peptide biotinylé, selon l'une quelconque des revendications 1 à 3, ou une composition peptidique, selon l'une quelconque des revendications 4 à 5.

25

```
         10          20          30          40          50          60
          |           |           |           |           |           |
GIGLVIVLAIMAIIAAAGAGLGVANAVQQSYTRTAVQSLANATAAQQNVLEATYAMVQHVAK

         70          80          90         100         110         120
          |           |           |           |           |           |
GVRILEARVARVEVLVDRMMVYQELDCWHYQHYCVTSTKSEVAKYVNWTRYRDNCTWQQWEE

        130         140         150         160         170         180
          |           |           |           |           |           |
EIEQHEANLSQLLKEAALQVHIAQRDAQRVPDVWTALQEAFDWAVWFAWLKYIPWIVIPIIG

        190         200         210         220         230         240         250
          |           |           |           |           |           |           |
LICFRIIMCVVSMCLQAYKQTTADQIYTGNSGDRRTSGPRGKTRRRKDGLSGFEDLKQEKRTSR

        260         270         280         290         300         310
          |           |           |           |           |           |
RSFTQIWRATWQAWKNSPWRKSWRRSLYMSLLPLLAVQQWLEETGWIGGNQLKNKKERVDCQ

        320
          |
DREQMPMLGNDYVEMK
```

Figure 1

```
           10          20          30          40          50          60
           |           |           |           |           |           |
MAHHHHHHVDDDDKGGSIEGRIVNLQAGGRSWKAVDSVVFQQLQTVAMQHGLVSEDFERQLA


           70          80          90         100         110         120
           |           |           |           |           |           |
YYATTWTSKDILEVLAMMPGNRAQKELIQGKLNEEAERWVRQNPPGPNVLTVDQIMGVGQTN


          130         140         150         160         170         180
           |           |           |           |           |           |
QQASQANMDQRRELCLQWVITALRAVRHMSHRPGNPMLVKQKNTESYEDFIARLLEAIDAEP


          190         200         210         220         230
           |           |           |           |           |
VTDPIKTYFKSDSAYTNASTDCQKQMDRVLGNRVQQASVEEKMQACRDVGSEG
```

(SEQ ID No 88)

Figure 2

Figure 3

Figure 4

Figure 5

# ELISA MVV gp46 TM1

Direct coating serumdilution 1/500

Fig 6 A

* : negative sera

# ELISA MVV gp46 TM1
## Streptavidin coating/ serumdilution 1/500

O.D.

coating
■Streptavid+ TM1 ■SA control ✿c.o.

gp135 +    gp135 +/-    p25 +    doubtful

*:negative sera

Fig 6b

EP 1 073 676 B1

# MVV TM peptides

MVV sera 1/500

Figure 7

# MVV p25

MVV sera 1/500
Figure 8

# MVV combi complex

S/N

MVV sera 1/500

Figure 9